# EUROPEAN PATENT SPECIFICATION

(11) **EP 1 781 811 B1**
(45) Date of publication and mention of the grant of the patent: **23.06.2010**
(21) Application number: 05761958.7
(22) Date of filing: 30.06.2005
(51) Int. Cl.: C12Q 1/68

(54) **METHOD OF DETECTING MUTATIONS IN THE GENE ENCODING CYTOCHROME P450-2D6**
VERFAHREN ZUM NACHWEIS VON MUTATIONEN IM FÜR CYTOCHROM P450-2D6 CODIERENDEN GEN
PROCEDE PERMETTANT DE DETECTER DES MUTATIONS DANS LE GENE CODANT LE CYTOCHROME P450-2D6

(30) Priority: 30.06.2004 US 583605 P
(43) Date of publication of application: 09.05.2007
(73) Proprietor: Luminex Molecular Diagnostics, Inc., Toronto, ON M5G 1Y8 (CA)
(72) Inventor: MERANTE, Frank, Etobicoke, Ontario M8Y 3H1 (CA); GORDON, James D., Mississauga, Ontario L5E 3E2 (CA); BORTOLIN, Susan, Oakville, Ontario L6H 6M8 (CA)
(74) Representative: Williams, Aylsa
(86) International application number: PCT/CA2005/001000
(87) International publication number: WO 2006/002526

(56) References cited:
- WO-A-01/29260
- WO-A1-00/47766
- WO-A1-91/10745
- WO-A1-03/050282
- WO-A2-01/55432
- WO-A2-02/059355
- WO-A2-02/083839
- JP-A- 2002 119 299
- US-A1- 2004 091 909
- US-A1- 2004 091 909
- US-A1- 2004 096 874
- DATABASE GENBANK [Online] 22 November 1994 (1994-11-22), KIMURA ET AL.: "Human cytochrome P450 IID6 (CYP2D6) gene, complete cds" XP002458244 retrieved from NCBI Database accession no. M33388
- CYTOCHROME (CYP) P450 ALLELE NOMENCLATURE COMMITTEE: "CYP2D6 allele nomenclature" INTERNET ARTICLE, [Online] 29 August 2007 (2007-08-29), XP002458243 Retrieved from the Internet: URL:http://www.cypalleles.ki.se/cyp2d6.htm > [retrieved on 2007-11-12]
- DALY A.K. ET AL: 'NOMENCLATURE FOR HUMAN CYP2D6 ALLELES' PHARMACOGENETICS vol. 6, no. 3, June 1996, pages 193 - 201, XP001018203

## Description

### BACKGROUND OF THE INVENTION

### FIELD OF THE INVENTION

The present invention relates to methods and kits for the detection of mutations located in the gene encoding Cytochrome P450-2D6.

### DESCRIPTION OF THE PRIOR ART

Enzymes within the cytochrome P450 family are involved in the phase one metabolism of a wide range of small molecules including a large number of prescription drugs. CYP2D6 (Debrisoquine-4-hydroxylase) is involved in the metabolism of at least 58 clinically relevant drugs. Drug metabolism defects resulting from mutations in CYP2D6 can cause the accumulation of drugs to toxic levels thereby contributing to potential adverse drug reactions (ADRs).

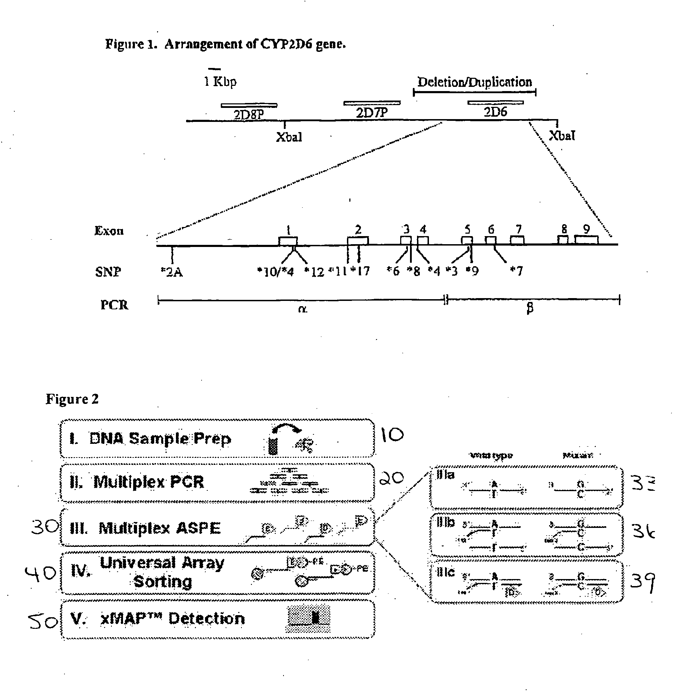

The gene encoding cytochrome P450 2D6 is located on chromosome 22q13.1. Two spliced pseudogenes (CYP2D7P and CYP2D8P) that show a high degree of DNA sequence homology with CYP2D6 lie immediately upstream of CYP2D6 as shown in Figure 1. The gene consists of nine exons arranged in a region of approximately 4.5 Kbp. The gene encodes the enzyme debrisoquine 4-hydroxylase. Figure 1 shows the arrangement of the CYP2D6 gene and its relation to the two pseudogenes as well as a region deleted or duplicated in the major genomic variations.

A consensus nomenclature for Cytochrome P450 genes has been described by Daly et al. (1996). According to this nomenclature, alleles are denoted CYP plus the gene name (i.e. CYP2D6) followed by an asterisk and then the mutation as an Arabic numeral. For the most part the different CYP2D6 alleles are haplotypes consisting of a number of point mutations or small variants occurring together within the same gene. Subtypes of the same allele are further designated by letters (i.e. CYP2D6*4A). The mutations described for CYP2D6 are listed in the official homepage for the Human P450 Allele Nomenclature Committee (http://www.imm.ki.se/CYPalleles/default.htm). Numbering of nucleotide positions in the database is based on their position in the genomic entry (Genbank accession # M33388 or SEQ ID No: 1) but the numbering starts at the A in the ATG rather than using the number in the Genbank entry.

Genetic testing can be used to identify individuals at risk for ADRs based on their genetic profile and allow physicians to alter dosing regimens or choose alternate drugs to reduce the potential risk of an ADR. A need exists, however, for a rapid, and accurate test for the detection of specific mutations in the gene encoding CYP2D6.

**Multiplex Allele Specific Primer Extension and Solid Support Detection of Mutations**

Multiplex allele specific primer extension, and hybridization of extended primers to a solid support is described in the prior art. ASPE technology has been generally described in U.S. Patent No. 4,851,331. The technology is designed to identify the presence or absence of specific polymorphic sites in the genome.

Multiplex ASPE in conjunction with hybridization to a support for mutation detection can be described generally as follows:

Amplifying regions of DNA comprising polymorphic loci utilizing a multiplexed, PCR.

2) Allele specific extension of primers wherein the amplified regions of DNA serve as target sequences for the allele specific extension. Extension primers that possess a 3' terminal nucleotide which form a perfect match with the target sequence are extended to form extension products. Modified nucleotides are incorporated into the extension product, such nucleotides effectively labelling the extension products for detection purposes. Alternatively, an extension primer may instead comprise a 3' terminal nucleotide which forms a mismatch with the target sequence. In this instance, primer extension does not occur.

3) Hybridizing the extension product to a probe on a solid support, such as a microarray, wherein the probe is complementary to the 5' end of the extension product.

The extension primers used in a methodology as described above, possess unique sequence tags at their 5' ends. For example, the sequence tags may allow the extension products to be captured on a solid support.

Variations of the above technology have been described, for example, in U.S. Patent No. 6,287,778 (WO 01/29260) and PCT Application (WO 00/47766).
In addition, US 2004/0091909 discloses a method for the rapid and simultaneous screening of large numbers of samples of several polymorphisms of a cytochrome P450 enzyme.

It is an object of the present invention provide a cost effective, fast, and accurate method for identifying variants in the gene encoding CYP2D6.

### SUMMARY OF THE INVENTION

In one embodiment, the present invention provides a method for detecting in a sample the presence or absence of nucleotide variants at polymorphic sites in the gene encoding cytochrome P450-2D6, the method comprising the steps of;
a) amplifying from the sample regions of DNA containing the variants to form amplified DNA products;
b) hybridizing at least two tagged allele specific extension primers to a complementary target sequence in the amplified DNA products, wherein each tagged allele specific extension primer has a 3'-end hybridizing portion capable of hybridizing to the complementary amplified DNA, and wherein the 3' end hybridizing portions of the at least two tagged allele specific extension primers each consist of a sequence selected from the group consisting of bases from position 25 to the 3' terminal nucleotide of SEQ ID NO: 10 to SEQ ID NO: 35, and a 5'-end tag portion complementary to a corresponding anti-tag sequence, the terminal nucleotide of the 3' end hybridizing portion being either complementary to a suspected variant nucleotide or to the corresponding wild type nucleotide of the site;
c) extending the at least two tagged allele specific extension primers, using labelled nucleotides, if the terminal nucleotide of each 3' end hybridizing portion is a perfect match to the complementary amplified DNA product;
d) hybridizing the at least two tagged allele specific extension primers to their corresponding anti-tag sequences and detecting the presence of labelled extension products.

In another embodiment, the present invention provides, a method for detecting the presence or absence of nucleotide variants at polymorphic sites in the gene encoding cytochrome P450-2D6, said variants selected from the group of variants listed in table 1, the method comprising the steps of;

a) amplifying regions of DNA containing the variants to form amplified DNA products;

b) hybridizing at least two tagged allele specific extension primers to a complementary target sequence in the amplified DNA products, wherein the at least two tagged allele-specific extension primers are selected from the group consisting of SEQ ID NO: 10 to SEQ ID NO: 35, each tagged allele specific extension primer having a 3'-end hybridizing portion capable of hybridizing to the amplified DNA, and a 5'-end tag portion complementary to a corresponding probe sequence, the terminal nucleotide of the 3' end hybridizing portion being either complementary to a suspected variant nucleotide or to the corresponding wild type nucleotide of the site;

c) extending the at least two tagged allele specific extension primers, using labelled nucleotides, if the terminal nucleotide of the 3' end hybridizing portion is a perfect match to an allele of one of the polymorphic sites in the amplified DNA products;

d) hybridizing the at least two tagged allele specific extension primers to the corresponding probe sequence and detecting the presence of labelled extension products.

In another embodiment, the present invention provides a kit for detecting the presence or absence of nucleotide variants at polymorphic sites in the gene encoding cytochrome P450-2D6, said variants selected from the group of variants listed in table 1, said kit comprising a set of at least two tagged allele specific extension primers wherein each tagged allele specific extension primer has a 3'-end hybridizing portion including a 3' terminal nucleotide being either complementary to a suspected variant nucleotide or to the corresponding wild type nucleotide of one of the polymorphic sites and a 5'-end tag portion complementary to a corresponding probe sequence, and wherein the at least two tagged allele-specific extension primers are selected from the group consisting of SEQ ID NO: 10 to SEQ ID NO: 35.

Also disclosed is a kit for detecting the presence or absence of nucleotide variants at polymorphic sites in the gene encoding cytochrome P450-2D6, said variants selected from the group of variants listed in table 1, said kit comprising a set of PCR amplification primers for amplifying regions of DNA containing the at least two polymorphic sites, said set comprising at least two pairs of PCR primers selected from the group of pairs consisting of:
SEQ ID NO: 2 and SEQ ID NO: 3, SEQ ID NO: 4 and SEQ ID NO: 5, SEQ ID NO: 6 and SEQ
ID NO: 7, and SEQ, ID NO: 8 and SEQ ID NO: 9.

### BRIEF DESCRIPTION OF THE DRAWINGS

These and other features of the preferred embodiments of the invention will become more apparent in the following detailed description in which reference is made to the appended drawings wherein:

Figure 1 depicts the arrangement of the CYP2D6 gene.

Figure 2 depicts a general overview of steps of the present invention. '

Figures 3 to 7 present genotyping results obtained using the method of the present invention.

### DESCRIPTION OF THE PREFERRED EMBODIMENTS

The following terms used herein will be understood to have the meanings defined below.

The term "mutations" as used herein refers to a number of classes of alteration in a nucleotide sequence including but not limited to the eight single base pair substitutions, two single base pair deletions, and one, three base pair deletion identified by the method of the present invention.

The terms "oligonucleotide" and "polynucleotide" as used herein refer to DNA sequences being of greater than one nucleotide in length. Such sequences may exist in either single or double-stranded form. Examples of oligonucleotides described herein include PCR primers, ASPE primers, and anti-tags.

The term "allele" is used herein to refer to variants of a nucleotide sequence.

The expression "allele specific primer extension (ASPE)", as used herein, refers to a mutation detection method utilizing primers which hybridize to a corresponding DNA sequence and which are extended depending on the successful hybridization of the 3' terminal nucleotide of such primer. Amplified regions of DNA serve as target sequences for ASPE primers. ASPE primers include a 3' end-hybridizing portion which hybridizes to the amplified regions of DNA. ASPE primers that possess a 3' terminal nucleotide which form a perfect match with the target sequence are extended to form extension products. Modified nucleotides can be incorporated into the extension product, such nucleotides effectively labelling the extension products for detection purposes. Alternatively, an extension primer may instead comprise a 3' terminal nucleotide which forms a mismatch with the target sequence. In this instance, primer extension does not occur unless the polymerase used for extension inadvertently possesses exonuclease activity or is prone to misincorporation.

The term "genotype" refers to the genetic constitution of an organism. More specifically, the term refers to the identity of alleles present in an individual. "Genotyping" of an individual or a DNA sample refers to identifying the nature, in terms of nucleotide base, of the two alleles possessed by an individual at a known polymorphic site.

The term "polymorphism", as used herein, refers to the coexistence of more than one form of a gene or portion thereof.

The term "PCR", as used herein, refers to the polymerase chain reaction. PCR is a method of amplifying a DNA base sequence using a heat stable polymerase and a pair of primers, one primer complementary to the (+)-strand at one end of the sequence to be amplified and the other primer complementary to the (-) strand at the other end of the sequence to be amplified. Newly synthesized DNA strands can subsequently serve as templates for the same primer sequences and successive rounds of heat denaturation, primer annealing and strand elongation results in rapid and highly specific amplification of the desired sequence. PCR can be used to detect the existence of a defined sequence in a DNA sample.

The term "primer", as used herein, refers to a short single-stranded oligonucleotide capable of hybridizing to a complementary sequence in a DNA sample. A primer serves as an initiation point for template dependent DNA synthesis. Deoxyribonucleotides can be joined to a primer by a DNA polymerase. A "primer pair" or "primer set" refers to a set of primers including a 5'upstream primer that hybridizes with the complement of the 5' end of the DNA sequence to be amplified and a 3' downstream primer that hybridizes with the 3' end of the DNA sequence to be amplified. The term "PCR primer" as used herein refers to a primer used for a PCR reaction: The term "ASPE primer" as used herein refers to a primer used for an ASPE reaction.

The term "tag" as used herein refers to an oligonucleotide sequence that is coupled to an ASPE primer. The sequence is generally unique and non-complementary to the human genome while being substantially complementary to a probe sequence. The probe sequence may be, for example, attached to a solid support. Tags serve to bind the ASPE primers to a probe.

The term "tagged ASPE primer" as used herein refers to an ASPE primer that is coupled to a tag.

The term "anti-tag" or "probe" as used herein refers to an oligonucleotide sequence having a sequence complementary to, and capable of hybridizing to, the tag sequence of an ASPE primer. The "anti-tag" may be coupled to a support.

The term "wild type" or "wt" as used herein refers to the normal, or non-mutated, or functional form of a gene.

The term "homozygous wild-type" as used herein refers to an individual possessing two copies of the same allele, such allele characterized as being the normal and functional form of a gene.

The term "heterozygous" or "HET" as used herein refers to an individual possessing two different alleles of the same gene.

The term "homozygous mutant" as used herein refers to an individual possessing two copies of the same allele, such allele characterized as the mutant form of a gene.

The term "mutant" as used herein refers to a mutated, or potentially non-functional form of a gene.

The term "deletion" as used herein refers to a mutation in which a portion of genomic DNA is deleted from a gene. The deletion may serve to eliminate all enzyme activity contributed by the chromosome where the deletion is located.

The term "duplication" as used herein refers to a mutation in which multiple copies of a gene may be present on an affected chromosome. The duplication may result in overproduction of an enzyme due to the presence of multiple copies of a specific gene.

The present invention was developed in response to a need for a rapid, highly specific, and cost-effective method to genotype individuals susceptible to adverse drug reactions. More specifically, a method for identifying individuals who may have drug metabolism defects resulting from mutations in the CYP2D6 gene is disclosed.

The present invention provides a novel, multiplex method of detecting multiple mutations located in the gene encoding CYP2D6. Specifically, the methodology can be used for the detection of the presence or absence of two or more mutations selected from the group consisting of the mutations identified in Table 1. In a preferred embodiment, the present invention provides a method of detecting the presence or absence of all the mutations identified in Table 1.

**Table 1: Mutations of the Gene Encoding CYP2D6**

| ***Variant*** | ***Major Allele*** †**-** | ***Effect*** | ***PCR Amplimer*** |
|---|---|---|---|
| / | *Gene* | ↑ *Gene* | α |
| | *Duplication* | *Copy* | |
| / | **5* | *Gene* | β |
| | | *Deletion* | |
| *-1584C>G* | **2A* | *Expression* | α |
| | *Promoter* | | |
| *100C>T* | **4(A-L)* | *P34S* | α |
| | **10(A,B)* | | |
| *124G>A* | **12* | *G42R* | α |
| *883G>C* | *11 | *Splicing* | α |
| *1023C>T* | *17 | *T1071* | α |
| *1707T>del* | **6(A-D)* | *Frameshift* | α |
| *1758G>T* | **8* | *Stop* | α |
| | | *Codon* | |
| *1846G>A* | **4(A-L)* | *Splicing* | α |
| *2549A>del* | **3(A,B)* | *Frameshift* | β |
| *2613-2615* | *9 | *K281*Δ | β |
| *delAGA* | | | |
| *2850C>T* | **2, *17* | *R296C* | β |
| *2935A>C* | **7* | *H324P* | β |

| | | | |
|---|---|---|---|
| ↑*For *4, no *4I designation exists* | | | |

The positive detection of one or more of the mutations identified in Table 1 may be indicative of an individual having a predisposition to adverse drug reactions.

Figure 1 shows the location of the variants assayed for in the method of the present invention and the two coding region PCR amplimers used in the method (discussed further below).

The present invention is further characterized by a high level of specificity. Such specificity is required in order to ensure that any result generated is a true representation of the genomic target and not simply the result of non-specific interactions occurring between reagents present in reactions. This is especially important for multiplexed DNA-based tests where the numerous sequences present in the reaction mixture, most of which are non-complementary, may interact non-specifically depending on the reaction conditions.

The methodology of the present invention utilizes the combination of multiplex ASPE technology with hybridization of tagged and labelled extension products to probes in order to facilitate detection. Such methodology is suitable for high-throughput clinical genotyping applications.

In one embodiment, the present invention provides a method for detecting in a sample the presence or absence of nucleotide variants at polymorphic sites in the gene encoding cytochrome P450-2D6, the method comprising the steps of;
a) amplifying from the sample regions of DNA containing the variants to form amplified DNA products;
b) hybridizing at least two tagged allele specific extension primers to a complementary target sequence in the amplified DNA products, wherein each tagged allele specific extension primer has a 3'-end hybridizing portion capable of hybridizing to the complementary amplified DNA, and wherein the 3' end hybridizing portions of the at least two tagged allele specific extension primers each consist of a sequence selected from the group consisting of bases from position 25 to the 3' terminal nucleotide of SEQ ID NO: 10 to SEQ ID NO: 35, and a 5'-end tag portion complementary to a corresponding anti-tag sequence, the terminal nucleotide of the 3' end hybridizing portion being either complementary to a suspected variant nucleotide or to the corresponding wild type nucleotide of the site;
c) extending the at least two tagged allele specific extension primers, using labelled nucleotides, if the terminal nucleotide of each 3' end hybridizing portion is a perfect match to the complementary amplified DNA product;
d) hybridizing the at least two tagged allele specific extension primers to their corresponding anti-tag sequences and detecting the presence of labelled extension products. Detection of a labelled extension product is indicative of the presence of the allele complementary to the 3'-terminal nucleotide of the ASPE primer. In the absence of a labelled extension product, it is determined that the allele corresponding to the 3' end of the ASPE primer is not present in the sample.

A general overview of one example of the above-mentioned method is presented in figure 2. A DNA sample is first prepared **10** using methods known in the art. Multiplex PCR amplification **20** is conducted in order amplify regions of DNA containing variant sites in the gene encoding cytochrome P450-2D6. A multiplex ASPE reaction **30** is then conducted. By example only, **33** illustrates a wild type and a mutant allele of a gene. At step **36** ASPE primers are hybridized to amplified regions of DNA. If the 3' terminal nucleotide of an ASPE primer is complementary to a corresponding nucleotide in the target sequence, a labelled extension product is formed **39** as will be described further below. The ASPE may be sorted on an addressable universal sorting array **40** wherein the presence of a labelled extension product may be detected using, for example, xMAP detection **50.**

Figure 1 shows the location of the variants assayed for in the method of the present invention, and the two coding region PCR amplimers used in the assay.

In addition to the small nucleotide variantions listed in Table 2., two large genomic rearrangements also affect CYP2D6 activity. The CYP2D6*5 allele is characterized by the deletion of 12.1 Kbp of genomic DNA including the entire 2D6 Gene. This deletion has a null phenotype eliminating all enzyme activity contributed by the chromosome on which it lies.

The CYP2D6xn allele is characterized by the tandem duplication of the 2D6 gene. The repeated unit is roughly 12.1 Kbp in length and is, in fact, identical to the region deleted in 2D6*5. In most cases the duplication results in the presence of two copies of the 2D6 gene on the affected chromosome although it has been observed that in rare cases up to 13 duplicated copies can be present. Both the original and duplicated copies of 2D6 are transcriptionally and translationally active resulting in overproduction of the enzyme and an ultra metabolizer phenotype (greater than normal levels of CYP2D6 activity). Most occurrences of the 2D6xn duplication have been observed with the 2D6*1 or 2D6*2 genotypes which are associated with the extensive metabolizer phenotype although the duplication has also been seen in association with 2D6*4 genotype.

### DNA Sample Preparation

Patient samples can be extracted with a variety of methods known in the art to provide nucleic acid (most preferably genomic DNA) for use in the following method.

### Amplification

In a first step, at least one region of DNA from the gene encoding CYP2D6 is amplified. The at least one region amplified contains mutation sites listed in table 1.

In a preferred embodiment of the present invention, PCR amplification of regions containing mutation sites in the gene encoding CYP2D6 is initiated using PCR primer pairs selected from the group of primer pairs consisting of: SEQ ID NO.: 2 and SEQ ID NO.: 3, SEQ ID NO.: 4 and SEQ ID NO.: 5, SEQ ID NO.: 6 and SEQ ID NO.: 7, and SEQ ID NO.: 8 and

### SEQ ID NO.: 9.

The relationships of each pair of primers to the mutations listed in Table 1 and to the detection of the deletion and duplication regions described above is presented in Table 2.

**Table 2: Primer Pairs Used to Amplify Regions Containing CYP2D6 Mutations**

| **PCR Primer Pair** | **Mutations Contained in Amplimer** |
|---|---|
| SEQ ID NO: 2 and 3 | All mutations (α-primers) |
| SEQ ID NO: 4 and 5 | Duplication Regions |
| SEQ ID NO: 6 and 7 | All mutations (β-primers) |
| SEQ ID NO: 8 and 9 | Deletion Regions |

An individual skilled in the art will recognize that alternate PCR primers could be used to amplify the target polymorphic regions, and deletion and duplication regions, however, in a preferred embodiment the primers listed in Table 2 are selected due to their minimal non-specific interaction with other sequences in the reaction mixture.

The presence of the deletion and duplication genomic rearrangements are detected by a PCR based mechanism. These approaches are adapted from the work of Steen *et al.,* (1995) for the detection of 2D6*5 (deletion) and Løvlie *et al.,* (1996) for the detection of the 2D6xn (duplication). The basis of these approaches is to utilize a pair of PCR primers that will yield a product only when the particular genomic rearrangement (deletion or duplication) is present. Since the recombination event in both the deletion and duplication occurs with a 2.8 Kbp. repeat region, the PCR amplimer must span this region. Thus, the deletion, when present, would generate a product of 3.5 Kb. While the duplication, when present, would generate a product of 3.2 Kb. The deletion and duplication primers cannot be used in the same PCR reaction since the mixing of primers would generate a signal from all WT genomic DNA samples. To minimize the number of PCR reactions performed the megaplex reactions are multiplexed by combining the α-primers with the duplication primers (the α reaction set) and the β-primers with the duplication primers (the β reaction set), as outlined in the example provided below.

### ASPE

The ASPE step of the method of the present invention is conducted using tagged ASPE primers selected from the group of ASPE primers consisting of SEQ ID NO: 10 to SEQ ID NO.: 35.

The ASPE primer set of the present invention has been optimized to ensure high specificity and accuracy of diagnostic tests utilizing such allele specific primers.

Table 3 presents a listing of the ASPE primers used in a preferred embodiment of the present invention. The suffix "wt" indicates an ASPE primer used to detect the wild type form of the gene encoding CYP2D6 at a specific mutation site. The suffix "mut" indicates an ASPE primer used to detect a mutant form of the gene encoding CYP2D6 at a specific mutation site. The suffix "dup" indicates an ASPE primer used to detect a duplication region. The suffix "del" indicates an ASPE primer used to detect a deleted region. Bases 1 to 24 of each of SEQ ID NO.: 10 to SEQ ID NO: 35 are the 5' portions of the ASPE primers that are complementary to specific probe sequences. Although the specific sequences listed in table 3 are preferred, in alternate embodiments of the present invention, it is possible to combine different 5' portions of the sequences in Table 3 (bases 1 to 24 of SEQ ID NOs: 10 to 35) with different 3' end hybridizing portions of the sequences in Table 3 (bases 25 and up of SEQ ID NOs: 10 to 35).

The orientation of each of the ASPE primers is also presented in Table 3.

**Table 3: P450-2D6 ASPE Primer Sequences**

| **SEQ ID NO :** | **Site Detected** | **Direction** |
|---|---|---|
| 10 | SNP17promo-wt | Forward |
| 11 | L-SNP17promo-mut | Forward |
| 12 | SNP13*3wt | Forward |
| 13 | SNP13*3mut | Forward |
| 14 | SNP12*4wt | Reverse |
| 15 | SNP12*4mut | Reverse |
| 16 | SNP1*10wt | Forward |
| 17 | SNP1*10mut | Forward |
| 18 | SNP10*6wt | Forward |
| 19 | L-SNP10*6mut | Forward |
| 20 | SNP15*7wt | Forward |
| 21 | SNP15*7mut | Forward |
| 22 | SNP11*8wt | Forward |
| 23 | SNP11*8mut | Forward |
| 24 | SNP3*11wt | Forward |
| 25 | SNP3*11mut | Forward |
| 26 | SNP2*12wt | Reverse |
| 27 | SNP2*12mut | Reverse |
| 28 | SNP14*17wt | Forward |
| 29 | S2-SNP14*17 | Forward |
| 30 | S-SNP20*9wt | Reverse |
| 31 | S-SNP20*9mut | Reverse |
| 32 | Sdup | Forward |
| 33 | S*5del | Forward |
| 34 | L1*17(1023wt) | Forward |
| 35 | S1*17(1023mut) | Forward |

The 3' end hybridizing portion of the extension primer is hybridized to the amplified material. Where the 3' terminal nucleotide of the 3' end hybridizing portion of the ASPE primer is complementary to the polymorphic site, primer extension is carried out using modified nucleotides. Where the 3' terminal nucleotide of the ASPE primer is not complementary to the polymorphic region, no primer extension occurs.

In one aspect, labelling of the extension products is accomplished through the incorporation of biotinylated nucleotides into the extension product which may be identified using fluorescent (Streptavidin-Phycoerythrin) or chemiluminescent (Streptavidin-Horseradish Peroxidase) reactions. However, an individual skilled in the art will recognize that other labelling techniques may be utilized. Examples of labels useful for detection include but are not limited to radiolabels, fluorescent labels (e.g fluorescein and rhodamine), nuclear magnetic resonance active labels, positron emitting isotopes detectable by a positron emission tomography ("PET") scanner, and chemiluminescers such as luciferin, and enzymatic markers such as peroxidase or phosphatase.

Each ASPE primer used in the methodology as described above, possess a unique sequence tag at their 5' ends. The sequence tags allow extension products to be detected with a high degree of specificity, for example, through capture on a solid support in order to facilitate detection.

**Detection**

The tagged 5' portions of the allele specific primers of the present invention are complementary to probe sequences. Upon hybridization of the allele specific primers to a corresponding probe sequence the presence of extension products can be detected.

In a preferred embodiment, probes used in the methodology of the present invention are coupled to a solid support, for example a 'universal' bead-based microarray.

Examples of supports that can be used in the present invention include, but are not limited to, bead based microarrays and 2D glass microarrays. The preparation, use, and analysis of microarrays are well known to persons skilled in the art. (See, for example, Brennan, T. M. et al. (1995) U.S. Pat. No. 5,474,796; Schena, et al. (1996) Proc. Natl. Acad. Sci. 93:10614-10619; Baldeschweiler et al. (1995), PCT Application WO95/251116; Shalon, D. et al. (I 995) PCT application WO95/35505; Heller, R. A. et al. (1997) Proc. Natl. Acad. Sci. 94:2150-2155; and Heller, M. J. et al. (1997) U.S. Pat. No. 5,605,662.). Detection can be achieved through arrays using, for example, chemiluminescence or fluorescence technology for identifying the presence or absence of specific mutations.

Universal arrays function as sorting tools indirectly detecting the target of interest and are designed to be isothermal and minimally cross-hybridizing as a set. Examples of microarrays which can be used in the present invention include, but should not be limited to, Luminex's^{®} bead based microarray systems, and Metrigenix's^{™} Flow Thru chip technology.

In one aspect, for example, Luminex's 100 xMAP^{™} fluorescence based solid support microarray system is utilized. Anti-tag sequences complementary to the tag regions of the ASPE primers/extension products, described above, are coupled to the surface of internally fluorochrome-color-coded microspheres. An array of anti-tag microspheres is produced, each set of microspheres having its own characteristic spectral address. The mixture of tagged, extended, biotinylated ASPE primers is combined with the array of anti tagged microspheres and is allowed to hybridize under stringent conditions.

In a reaction mixture, a fluorescent reporter molecule (e.g. streptavidin-phycoerythrin) is used to detect labelled extension products which are synthesized when the terminal nucleotide of an ASPE primer is complementary to a corresponding nucleotide in the target sequence.

The reaction mixture, comprising microspheres, extension products etc. is injected into a reading instrument, for example Luminex's 100 xMAP^{™}, which uses microfluidics to align the microspheres in single file. Lasers are used to illuminate the fluorophores both internal to the microspheres, and attached to the surface in the form of extension products hybridized to anti-tag sequences. The Luminex 100 xMAP^{™}, interprets the signal received and identifies the presence of wild type and/or mutant alleles. The presence of the mutant allele of any one or more of the mutations presented in Table 2 may be indicative a predisposition for adverse drug reactions. Software can be provided which is designed to analyze data associated with the specific extension products and ariti-tagged microspheres of the present disclosure

In another aspect, the Metrigenix Flow-Thru three dimensional microchannel biochip (Cheek, B.J., Steel A.B., Torres, M.P., Yu, Y., and Yang H. Anal. Chem. 2001, 73, 5777-5783) is utilized for genotyping as known in the art. In this aspect, each set of microchannels represents a different universal anti-tag population. Anti-tag sequences corresponding to the tag regions of the ASPE primers/extension products, described above, are attached to the inner surface of multiple microchannels comprising a cell. Multiple cells make up a chip. The reaction mixture, including biotinylated extension products flows through the cells in the presence of a chemiluminescent reporter substrate such as streptavidin-horseradish peroxidase. Microarray chips can be imaged using technology known in the art, such as an ORCA-ER CCD (Hamamatsu Photonics K. K., Hamamatsu City, Japan), and imaging software, in order to identify the genotype of an individual.

### Kits

In an additional embodiment, the present invention provides kits for the multiplex detection of mutations in the gene encoding CYP2D6.

A kit that can be used for detection of the mutations of interest may contain the following components including: a PCR primer mix for amplifying regions containing mutation sites of interest (optionally including dNTPs), an ASPE primer mix for generation of labelled extension products (optionally including dNTPs) and a solid support, such as microarray beads, the beads having anti-tags complementary to the tagged regions of the ASPE primers. In addition, an individual skilled in the art would recognize other components which could be included in such kits including, for example, buffers and polymerases.

Kits of the present invention may include PCR primer pairs, ASPE primers, and tagged supports for all the mutations to be detected, or may be customized to best suit the needs of an individual end user. For example, if an end user wishes to detect only 5 of the mutations in the CYP2D6 gene, a kit can be customized to include only the PCR primer pairs, ASPE primers, and support required for the detection of the desired mutations. As such, the end user of the product can design a kit to match their specific requirements. In addition, the end user can also control the tests to be conducted at the software level when using, for example, a universal bead based-microarray for detection. For example, software can be provided with a kit, such software reading only the beads for the desired mutations or by reporting only the results from the desired mutation data. Similar control of data reporting by software can be obtained when the assay is performed on alternate platforms.

An individual skilled in the art will recognize that although the present method has been described in relation to the specific mutations identified in Table 1, PCR primers and ASPE primers used to detect additional mutations could be included in the above method and kits.

**EXAMPLE #1: ASPE/Microarray Detection of Mutations in the Gene Encoding CYP2D6**

The following represents an example protocol for use in the method of the present invention.

For each genomic sample being tested, two separate PCR reactions are performed. Each PCR reaction requires 25 ng genomic DNA (ie. 50 ng genomic DNA per sample). The first PCR (PCR-α) produces an alpha fragment (3.8 kb) (from PCR primer pair comprising SEQ ID NO: 2 and SEQ ID NO: 3) used to detect the variants shown in Table 1, as well as a duplication amplimer (3.2 kb) (from PCR primer pair comprising SEQ ID NO: 4 and SEQ ID NO: 5) which indicates the presence of the duplication genotype, if present. The second PCR (PCR-β) produces a beta fragment (2.6 kb)) (from PCR primer pair comprising SEQ ID NO: 6 and SEQ ID NO: 7)used to detect the variants shown in Table 2, as well as a deletion amplimer (3.5 kb) (from PCR primer pair comprising SEQ ID NO: 8 and SEQ ID NO: 9) indicative of the deletion genotype, if present. Following PCR amplification, the two reactions (PCR-α and PCR-β) are pooled. To enable efficient incorporation of biotin-dCTP during the Allele Specific Primer Extension (ASPE) reaction, the pooled PCR product is treated with Shrimp Alkaline Phosphatase (SAP) to inactivate any remaining nucleotides (particularly dCTP), and with Exonuclease I (EXO) to degrade any primers left over from the PCR reaction. ASPE is then carried out using 26 universally-tagged primers (SEQ ID NO: 10 to SEQ ID NO: 35) supplied in the ASPE primer mix. A 5 uL aliquot of the ASPE reaction is hybridized with the universal array (Bead Mix) in the presence of the hybridization buffer and incubated with Streptavidin, R-Phycoerythrin conjugate (reporter solution). Samples are read on the Luminex® 100 xMAP™ Instrument and signal is generated for each of the 12 small nucleotide variants as well as for the duplication and deletion amplimers (if present). These fluorescence values are then analyzed to determine whether the wild-type/mutant allele for each of the 12 small nucleotide variants has been detected or whether the samples carry an allele(s) with the deletion or duplication.

**Example #2: Detection of Mutations in the Gene Encoding CYP2D6**

**1) Oligonucleotides**

All oligonucleotides were synthesized by Integrated DNA Technologies (Coralville, IA). PCR primers were unmodified and were purified by standard desalting procedures. Universal anti-tags (probes) were 3'-C7 amino-modified for coupling to carboxylated microspheres. All anti-tags were reverse phase HPLC-purified. Chimeric ASPE primers which consisted of a 24mer universal tag sequence 5' to the allele-specific sequence were also unmodified but were purified by polyacrylamide gel electrophoresis. Following reconstitution, exact oligo concentrations were determined spectrophotometrically using extinction coefficients provided by the supplier. Reconstituted oligos were scanned between 200 and 500 nm and absorbance was measured at 260 nm to calculate oligo concentration.

**2) Reagents**

Expand Long Template PCR System was purchased from Roche Diagnostics (Indianapolis IN). Platinum Tsp DNA Polymerase, individual dNTPs and biotin-dCTP were purchased from Invitrogen Corporation (Carlsbad, CA). Shrimp alkaline phosphatase and Exonuclease I were purchased from USB Corporation (Cleveland, OH). Carboxylated fluorescent microspheres were provided by Luminex Corporation (Austin, TX). The EDC cross-linker (1-ethyl-3-(3-dimethylaminopropyl) carbodiimide hydrochloride) was purchased from Pierce (Rockford, IL). OmniPur reagents including MES (2-(N-morpholino)ethane sulfonic acid), 10% SDS, NaCl, Tris, Triton X-100, Tween-20 and TE buffer were purchased from EM Science (Darmstadt, Germany). The streptavidin-conjugated phycoerythrin was obtained from Molecular Probes Inc. (Eugene, OR).

**3) Genotyping**

a) MULTIPLEX PCR (two 2-plexes): Each PCR was carried out using 25 ng genomic DNA in a 10 µL final volume. A 'no target' PCR negative control was included with each assay run. The reaction consisted of 1x Expand Long Buffer 2, 200 umol/L each dNTP, 0.75 units of Expand Long Enzyme Mix (Roche), with primers ranging from 0.45 to 1umol/L. Samples were cycled in an MJ Research PTC-200 thermocycler (Waterdown MA) with cycling parameters set at 95°C for 3 minutes followed by 35 cycles at 95°C for 60 seconds, 66°C for 30 seconds and 72°C for 3 minutes. Samples were then held at 72°C for 5 minutes and kept at 4°C until use. Following completion of the PCR, the A and B reactions were pooled.

b) ALLELE-SPECIFIC PRIMER EXTENSION: Prior to the ASPE reaction, each pooled PCR reaction mixture was treated with shrimp alkaline phosphatase (SAP) to inactivate any remaining nucleotides (particularly dCTP) so that biotin-dCTP could be efficiently incorporated during the primer extension reaction. Each PCR reaction was also treated with exonuclease I (EXO) to degrade remaining PCR primers in order to avoid any interference with the tagged ASPE primers and the extension reaction itself. To each pooled sample (20µL, 2 µL SAP (= 2 units) and 0.5 µL EXO (= 5 units) were added directly to the sample. Samples were then incubated at 37°C for 30 minutes followed by a 15 minute incubation at 99°C to inactivate the enzymes. Samples were then added directly to the ASPE reaction.

Multiplex ASPE was carried out using 5 uL of treated PCR product in a final volume of 20 uL. Each reaction consisted of 20 mmol/L Tris-HCl pH 8.4, 50 mmol/L KCl, 1.25 mmol/L MgCl2, 5 umol/L biotin-dCTP, 5 umol/L each of dATP, dGTP and dTTP, 1.5 units Platinum Tsp DNA Polymerase and 2.5 nmol/L ASPE primer pool. The ASPE reactions were incubated at 96°C for 2 minutes and then subjected to 40 cycles at 94°C for 30 seconds, 54°C for 30 seconds and 74°C for 60 seconds. Reactions were then held at 4°C until use.

c) BEAD COUPLING: Amino-modified anti-tag sequences were coupled to carboxylated microspheres following Luminex's one-step carbodiimide coupling procedure. Briefly, 5 x 10⁶ microspheres were combined with 1 nmol NH₂-oligo in a final volume of 50 µL 0.1 mol/L MES, pH 4.5. A 10 mg/mL EDC working solution was prepared just prior to use and 2.5 µL was added to the bead mixture and incubated for 30 minutes. A second 2.5 µL aliquot of freshly prepared EDC was added followed by an additional 30 minute incubation. Following washes in 0.02% (v/v) Tween-20 and 0.1 % (w/v) SDS, the anti-tag coupled beads were resuspended in 100 uL TE buffer (10 mmol/L Tris, pH 8.0, 1 mmol/L EDTA). Bead concentrations were determined using a Beckman Coulter Z2 Particle Count and Size Analyzer (Coulter Corp, Miami FL).

d) UNIVERSAL ARRAY HYBRIDIZATION: Each hybridization reaction was carried out using approximately 2500 beads of each of the 26 anti-tag bearing bead populations. The beads were combined in hybridization buffer (0.22 mol/L NaCl, 0.11 mol/L Tris, pH 8.0 and 0.088% (v/v) Triton X-100) and 45 µL of the mix were added to each well of an MJ Research 96-well plate (Waterdown MA). A 5 µL aliquot of each ASPE reaction was then added directly to each well. The samples were then heated to 96°C for 1 minutes in an MJ Research PTC-200 followed by a one hour incubation at 37°C. Following this incubation, samples were filtered through a 1.2 um Durapore Membrane (Millipore Corp, Bedford, MA) and washed once using wash buffer (0.2 mol/L NaCl, 0.1 mol/L Tris, pH 8.0 and 0.08% (v/v) Triton X-100). The beads were then resuspended in 150 uL reporter solution (1 ug/mL streptavidin-conjugated phycoerythrin in wash buffer) and incubated for 15 minutes at room temperature. The reactions were read on the Luminex xMAP. Acquisition parameters were set to measure 100 events per bead population and a 100 uL sample volume. A gate setting was established prior to running the samples and maintained throughout the course of the study.

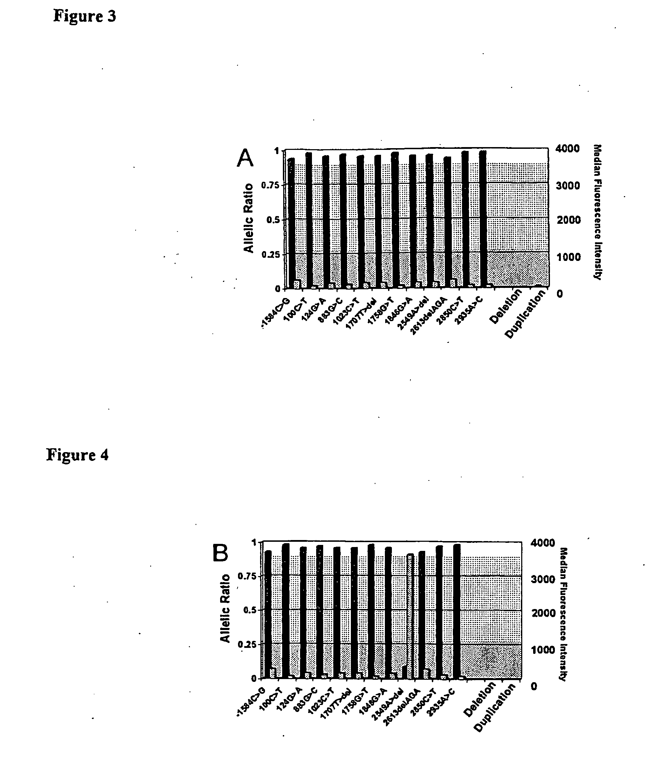

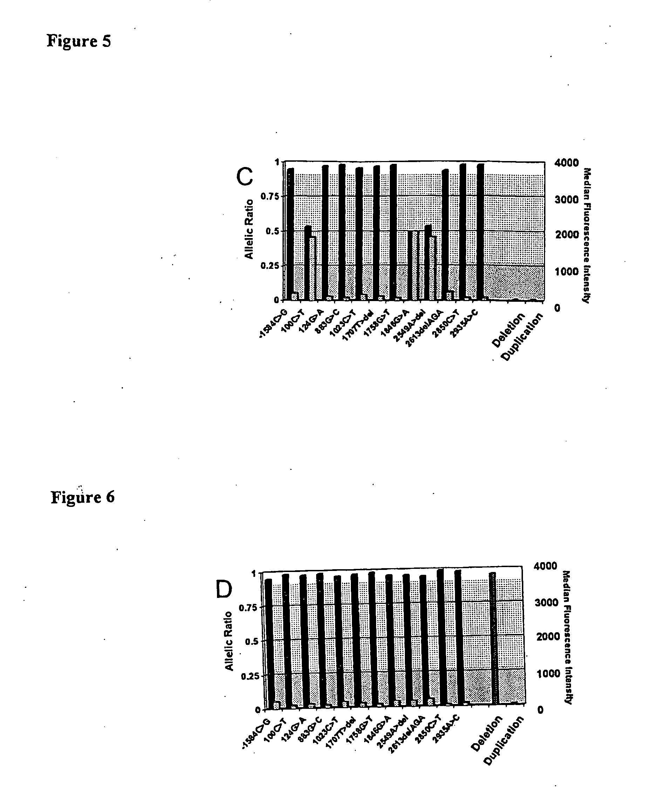

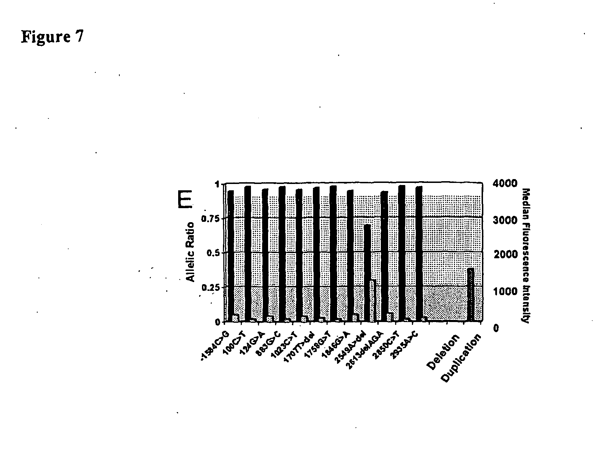

Representative results obtained with the kit of the present invention are presented in figures 3 to 7. WT (dark bars) and mutant (light bars) allelic ratios are shown for small nucleotide variations while median fluorescent intensity is shown for the deletion and duplication. Figure 3 shows results obtained for an individual who is WT for all alleles tested. Figure 4 shows the results obtained from an individual mutant for the 2549A>del (*3) variant. Figure 5 shows the result from an individual heterozygous for three variants; the 100C>T and 1846G>A variations are both found in the *4 allele. Figures 6 and 7 show the profiles seen with individuals with the gene deletion or duplication respectively.

## Claims

1. A method for detecting in a sample the presence or absence of nucleotide variants at polymorphic sites in the gene encoding cytochrome P450-2D6, the method comprising the steps of;
a) amplifying from the sample regions of DNA containing the variants to form amplified DNA products;
b) hybridizing at least two tagged allele specific extension primers to a complementary target sequence in the amplified DNA products, wherein each tagged allele specific extension primer has a 3'-end hybridizing portion capable of hybridizing to the complementary amplified DNA, and wherein the 3' end hybridizing portions of the at least two tagged allele specific extension primers each consist of a sequence selected from the group consisting of bases from position 25 to the 3' terminal nucleotide of SEQ ID NO: 10 to SEQ ID NO: 35, and a 5'-end tag portion complementary to a corresponding anti-tag sequence, the terminal nucleotide of the 3' end hybridizing portion being either complementary to a suspected variant nucleotide or to the corresponding wild type nucleotide of the site;
c) extending the at least two tagged allele specific extension primers, using labelled nucleotides, if the terminal nucleotide of each 3' end hybridizing portion is a perfect match to the complementary amplified DNA product;
d) hybridizing the at least two tagged allele specific extension primers to their corresponding anti-tag sequences and detecting the presence of labelled extension products.

2. The method of claim 1 wherein the 5'-end tag portions of the at least two tagged allele specific primers each comprise a sequence selected from the group consisting of bases I to 24 of SEQ ID NO: 10 to SEQ ID NO: 35 and wherein the sequence of each 5' end tag portion is unique.

3. The method of claim 1 wherein the anti-tag sequence is coupled to a solid support.

4. The method of claim 3 wherein the solid support is selected from the group consisting of beads, spectrally coded beads, and a chip based microarray.

5. The method of claim 1 wherein the step of amplifying is conducted by PCR using a set of PCR amplification primers, said set of PCR amplification primers comprising at least two pairs of PCR primers selected from the group of pairs consisting of:
SEQ ID NO: 2 and SEQ ID NO: 3, SEQ ID NO: 4 and SEQ ID NO: 5, SEQ ID NO: 6 and SEQ ID NO: 7, and SEQ ID NO: 8 and SEQ ID NO: 9, wherein the PCR primer pairs are selected according to the relationship of each primer pair to the selected variants to be detected.

6. The method according to claim 1, wherein the method comprising the steps of;
a) amplifying from the sample regions of DNA containing the selected variants to form amplified DNA products;
b) hybridizing at least two tagged allele specific extension primers to a complementary target sequence in the amplified DNA products, wherein the at least two tagged allele-specific extension primers are selected from the group consisting of SEQ ID NO: 10 to SEQ ID NO: 35, each tagged allele specific extension primer having a 3'-end hybridizing portion capable of hybridizing to the complementary amplified DNA, and a 5'-end tag portion complementary to a corresponding anti-tag sequence, the terminal nucleotide of the 3' end hybridizing portion being either complementary to a suspected variant nucleotide or to the corresponding wild type nucleotide of the site;
c) extending the at least two tagged allele specific extension primers, using labelled nucleotides, if the terminal nucleotide of each 3' end hybridizing portion is a perfect match to the complementary amplified DNA product;
d) hybridizing the at least two tagged allele specific extension primers to their corresponding anti-tag sequences and detecting the presence of labelled extension products.

7. The method of claim 6 wherein the anti-tag sequence is coupled to a solid support.

8. The method of claim 7 wherein the solid support is selected from the group consisting of beads, spectrally coded beads, and a chip based microarray.

9. The method of claim 6 wherein the step of amplifying is conducted by PCR using a set of PCR amplification primers, said set of PCR amplification primers comprising at least two pairs of PCR primers selected from the group of pairs consisting of:
SEQ ID NO: 2 and SEQ ID NO: 3, SEQ ID NO: 4 and SEQ ID NO: 5, SEQ ID NO: 6 and SEQ ID NO: 7, and SEQ ID NO: 8 and SEQ ID NO: 9, wherein the PCR primer pairs are selected according to the relationship of each primer pair to the selected variants to be detected.

10. A kit for detecting in a sample the presence or absence of nucleotide variants at polymorphic sites in the gene encoding cytochrome P450-2D6, said kit comprising a set of at least two tagged allele specific extension primers wherein each tagged allele specific extension primer has a 3'-end hybridizing portion including a 3' terminal nucleotide being either complementary to a variant nucleotide or to the corresponding wild type nucleotide of one of the polymorphic sites and a 5'-end tag portion complementary to a corresponding anti-tag sequence, and wherein the at least two tagged allele-specific extension primers are selected from the group consisting of SEQ ID NO: 10 to SEQ ID NO: 35.

11. The kit of claim 10 further comprising a set of PCR amplification primers for amplifying regions of DNA containing the polymorphic sites, said set of PCR amplification primers comprising at least two pairs of PCR primers selected from the group of pairs consisting of:
SEQ ID NO: 2 and SEQ ID NO: 3, SEQ ID NO: 4 and SEQ ID NO: 5, SEQ ID NO: 6 and SEQ ID NO: 7, and SEQ ID NO: 8 and SEQ ID NO: 9, wherein the PCR primer pairs are selected according to the relationship of each primer pair to the selected variants to be detected.

12. The kit of claim 10 further comprising a set of anti-tags, each anti-tag being complementary to nucleotides 1-24 of the selected at least two tagged allele-specific extension primers.

13. The kit of claim 12 wherein the anti-tags are coupled to a support.

## Patentansprüche

1. Verfahren zum Nachweis des Vorhandenseins oder der Abwesenheit von Nukleotidvarianten an polymorphen Stellen in dem Cytochrom P450-2D6 kodierenden Gen in einer Probe, wobei das Verfahren die folgenden Stufen umfasst:
a) Amplifizieren von DNA-Bereichen, die die Varianten enthalten, aus der Probe, um amplifizierte DNA-Produkte zu bilden,
b) Hybridisieren von wenigstens zwei markierten alielspezifischen Extensionsprimern an eine komplementäre Zielsequenz in den amplifizierten DNA-Produkten, wobei jeder markierte allelspezifische Extensionsprimer einen 3'-endständigen Hybridisierungsabschnitt aufweist, der in der Lage ist, an die komplementäre amplifizierte DNA zu binden, und wobei die 3'-endständigen Hybridisierungsabschnitte der wenigstens zwei markierten allelspezifischen Extensionsprimer jeweils aus einer Sequenz bestehen, die ausgewählt ist aus der Gruppe, bestehend aus Basen aus Position 25 bis zu dem 3'-endständigen Nukleotid von SEQ ID NO: 10 bis SEQ ID NO: 35, und einen 5'-endständigen Markierungsabschnitt, der komplementär zu einer korrespondierenden Anti-Markierungssequenz ist, wobei das endständige Nukleotid des 3'-endständigen Hybridierungsabschnitts entweder komplementär zu einem vermuteten abgewandelten Nukleotid ist oder zu dem entsprechenden Wildtyp-Nukleotid an der Stelle,
c) Verlängern der wenigstens zwei markierten allelspezifischen Extensionsprimer, unter Verwendung von markierten Nukleotiden, wenn das endständige Nukleotid von jedem 3'-endständigen Hybridisierungsabschnitt ein perfektes Gegenstück zu dem komplementären amplifizierten DNA-Produkt ist,
d) Hybridisieren der wenigstens zwei markierten allelspezifischen Extensionsprimer an ihre korrespondierenden Anti-Markierungssequenzen und Nachweisen des Vorhandenseins von markierten Verlängerungsprodukten.

2. Verfahren gemäß Anspruch 1, wobei die 5'-endständigen Markierungsabschnitte der wenigstens zwei markierten allelspezifischen Primer jeweils eine Sequenz umfassen, die ausgewählt ist aus der Gruppe, bestehend aus den Basen 1 bis 24 von SEQ ID NO: 10 bis SEQ ID NO: 35, und wobei die Sequenz jedes 5'-endständigen Markierungsabschnitts einzigartig ist.

3. Verfahren nach Anspruch 1, wobei die Anti-Markierungssectuenz an einen festen Träger gebunden ist.

4. Verfahren nach Anspruch 3, wobei der feste Träger ausgewählt ist aus der Gruppe, bestehend aus Perlen, spektralkodierten Perlen und einem Chip-basierten Mikroarray.

5. Verfahren nach Anspruch 1, wobei die Stufe des Amplifizierens durch PCR ausgeführt wird, unter Verwendung eines Sets von PCR-Verlängerungsprimern, wobei das Set von PCR-Verlängerungsprimern wenigstens zwei Paare von PCR-Primern umfasst, die ausgewählt sind aus der Gruppe von Paaren, bestehend aus: SEQ ID NO: 2 und SEQ ID NO: 3, SEQ ID NO: 4 und SET ID NO: 5, SEQ ID NO: 6 und SEC ID NO: 7 sowie SEQ ID NO: 8 und SEQ ID NO: 9, wobei die PCR-Primerpaare entsprechend der Verwandtschaft jedes Primerpaars zu den nachzuweisenden ausgewählten Varianten ausgewählt werden.

6. Verfahren gemäß Anspruch 1, wobei das Verfahren die folgenden Stufen umfasst:
a) Amplifizieren von DNA-Bereichen, die die ausgewählten Varianten enthalten, aus der Probe, um amplifizierte DNA-Produkte zu bilden,
b) Hybridisieren von wenigstens zwei markierten allelspezifischen Extensionsprimern an eine komplementäre Zielsequenz in den amplifizierten DNA-Produkten, wobei die wenigstens zwei allelspezifischen Extensionsprimer ausgewählt sind aus der Gruppe, bestehend aus SEQ ID NO: 10 bis SEQ ID NO: 35, wobei jeder markierte allelspezifische Extensionsprimer einen 3'-endständigen Hybridisierungsabschnitt hat, der in der Lage ist, an die komplementäre amplifizierte DNA zu hybridisieren, und einen 5'-endständigen Markierungsabschnitt, der komplementär zu einer entsprechenden Anti-Markierungssequenz ist, wobei das endständige Nukleotid des 3'-endständigen Hybridisierungsabschnitts entweder komplementär zu einem vermuteten abgewandelten Nukleotid oder zu dem korrespondierenden Wildtyp-Nukelotid an der Stelle ist,
c) Verlängern der wenigstens zwei markierten allelspezifischen Extensionsprimer, unter Verwendung von markierten Nukleotiden, wenn das endständige Nucleotid jedes 3'-endständigen Hybridisierungsabschnitts ein perfektes Gegenstück zu dem komplementären amplifizierten DNA-Produkt ist,
d) Hybridisieren der wenigstens zwei markierten allelspezifischen Extensionsprimer an ihre korrespondierenden Anti-Markierungssequenzen und Nachweisen des Vorhandenseins von markierten Verlängerungsprodukten.

7. Verfahren gemäß Anspruch 6, wobei die Anti-Markierungssequenz an einen festen Träger gebunden ist

8. Verfahren gemäß Anspruch 7, wobei der feste Träger ausgewählt ist aus der Gruppe bestehend aus Perlen, spektralkodierten Perlen und einem Chip-basiertem Mikroarray

9. Verfahren gemäß Anspruch 6, wobei die Stufe des Amplifizierens durch PCR ausgeführt wird, unter Verwendung eines Sets von PCR-Amplifikationsprimern, wobei das Set von PCR-Amplifilcationsprimern wenigstens zwei Paare von PCR-Primern umfasst, die ausgewählt sind aus der Gruppe von Paaren, bestehend aus: SEQ ID NO: 2 und SEQ ID NO: 3, SEQ ID NO: 4 und SEQ ID NO: 5, SEQ ID NO:6 und SEQ ID NO: 7 sowie SEQ ID NO: 8 und SEQ 10 NO:9, wobei die PCR-Primerpaare entsprechend der Verwandtschaft jedes Primerpaars zu den nachzuweisenden ausgewählten Varianten ausgewählt werden.

10. Kit zum Nachweis des Vorhandenseins oder der Abwesenheit von Nukleotidvarianten an polymorphen Stellen in dem Cytochrome P450-2D6 kodierenden Gen in einer Probe, wobei das Kit ein Set von wenigstens zwei markierten allelspezifischen Extensionsprimern umfasst, wobei jeder markierte allelspezifische Extensionsprimer einen 3'-endständigen Hybridisierungsabschnitt aufweist, der ein 3'-endständiges Nukleotid umfasst, das entweder komplementär zu einem abgewandelten Nukleotid oder zu dem entsprechenden Wildtyp-Nukleotid von einer der polymorphen Stellen ist, und einen 5'-endständigen Markierungsabschritt, der komplementär zu einer korrespondierenden Anti-Markierungssequenz ist, und wobei die wenigstens zwei markierten allelspezifischen Extensionsprimer ausgewählt sind aus der Gruppe, bestehend aus SEQ ID NO: 10 bis SEQ ID NO: 35

11. Kit gemäß Anspruch 10, welches weiter ein Set von PCR-Amplifikationsprimern zur Amplifizierung von die polymorphen Stellen enthaltenden DNA-Bereichen umfasst, wobei das Set von PCR-Amplifikationsprimern wenigstens zwei Paare von PCR-Primern umfasst, die ausgewählt sind aus der Gruppe von Paaren, bestehend aus: SEQ ID NO: 2 und SEQ ID NO: 3, SEQ ID NO: 4 und SEQ ID NO: 5, SEQ ID NO: 6 und SEQ ID NO: 7 sowie SEQ ID NO: 8 und SEQ ID NO: 9, wobei die PCR-Primerpaare entsprechend der Verwandtschaft jedes Primerpaars zu den nachzuweisenden ausgewählten Varianten ausgewählt sind

12. Kit gemäß Anspruch 10, welches weiter ein Set von Anti-Markierungen umfasst, wobei jede Anti-Markierung komplementär zu den Nukleotiden 1-24 der ausgewählten wenigstens zwei markierten allelspezifischen Extensionsprimern ist,

13. Kit gemäß Anspruch 12, wobei die Anti-Markierungen an einen Träger gebunden sind

## Revendications

1. Procédé permettant de détecter dans un échantillon la présence ou l'absence de variants nucléotidiques au niveau de sites polymorphes dans le gène codant pour le cytochrome P450-2D6, le procédé comprenant les étapes suivantes :
a) amplifier à partir de l'échantillon des régions d'ADN contenant les variants pour former des produits d'ADN amplifiés ;
b) hybrider au moins deux amorces d'extension marquées spécifiques d'allèle à une séquence cible complémentaire dans les produits d'ADN amplifiés, où chaque amorce d'extension marquée spécifique d'allèle a une partie hybridante à l'extrémité 3' capable de s'hybrider à l'ADN amplifié complémentaire, et où les parties hybridantes à l'extrémité 3' des au moins deux amorces d'extension marquées spécifiques d'allèle sont constituées chacune d'une séquence choisie dans le groupe constitué de bases depuis la position 25 jusqu'au nucléotide terminal en 3' de SEQ ID NO : 10 à SEQ ID NO : 35, et d'une partie marqueur à l'extrémité 5' complémentaire d'une séquence anti-marqueur correspondante, le nucléotide terminal de la partie hybridante à l'extrémité 3' étant soit complémentaire d'un nucléotide variant suspecté ou soit du nucléotide de type sauvage correspondant du site;
c) étendre les au moins deux amorces d'extension marquées spécifiques d'allèle, en utilisant des nucléotides marqués, si le nucléotide terminal de chaque partie hybridante à l'extrémité 3' présente un appariement parfait au produit d'ADN amplifié complémentaire ;
d) hybrider les au moins deux amorces d'extension marquées spécifiques d'allèle à leurs séquences anti-marqueur correspondantes et détecter la présence de produits d'extension marqués

2. Procédé selon la revendication 1, dans lequel les parties marqueurs à l'extrémité 5' des au moins deux amorces d'extension marquées spécifiques d'allèle comprennent chacune une séquence choisie dans le groupe constitué par les bases 1 à 24 de SEQ ID NO : 10 à SEQ ID NO : 35, et dans lequel la séquence de chaque partie marqueur à l'extrémité 5' est unique.

3. Procédé selon la revendication 1, dans lequel la séquence anti-marqueur est couplée à un support solide.

4. Procédé selon la revendication .3, dans lequel le support solide est choisi dans le groupe constitué par des billes, des billes à code spectral et un microréseau à base de puces.

5. Procédé selon la revendication 1, dans lequel l'étape d'amplification est conduite par PCR en utilisant un ensemble d'amorces d'amplification par PCR, ledit ensemble d'amorces d'amplification par PCR comprenant au moins deux paires d'amorces de PCR choisies dans le groupe des paires constitué par : SEQ ID NO : 2 et SEQ ID NO: 3, SEQ ID NO : 4 et SEQ ID NO : 5, SEQ ID NO : 6 et SEQ ID NO : 7, et SEQ ID NO : 8 et SEQ ID NO : 9, où les paires d'amorces de PCR sont choisies selon la relation de chaque paire d'amorces avec les variants sélectionnés à détecter.

6. Procédé selon la revendication 1, où le procédé comprenant les étapes suivantes :
a) amplifier à partir de l'échantillon des régions d'ADN contenant les variants sélectionnés pour former des produits d'ADN amplifiés ;
b) hybrider au moins deux amorces d'extension marquées spécifiques d'allèle à une séquence cible complémentaire dans les produits d'ADN amplifiés, où les au moins deux amorces d'extension marquées spécifiques d'allèle sont choisies dans le groupe constitué par : SEQ ID NO : 10 à SEQ ID NO : 35, chaque amorce d'extension marquée spécifique d'allèle ayant une partie hybridante à l'extrémité 3' capable de s'hybrider à l'ADN amplifié complémentaire, et une partie marqueur à l'extrémité 5' complémentaire d'une séquence anti-marqueur correspondante, le nucléotide terminal de la partie hybridante à l'extrémité 3' étant soit complémentaire d'un nucléotide variant suspecté ou soit du nucléotide de type sauvage correspondant du site ;
c) étendre les au moins deux amorces d'extension marquées spécifiques d'allèle, en utilisant des nucléotides marqués, si le nucléotide terminal de chaque partie hybridante à l'extrémité 3' présente un appariement parfait au produit d'ADN amplifié complémentaire;
d) hybrider les au moins deux amorces d'extension marquées spécifiques d'allèle à leurs séquences anti-marqueur correspondantes et détecter la présence de produits d'extension marqués.

7. Procédé selon la revendication 6, dans lequel la séquence anti-marqueur est couplée à un support solide.

8. Procédé selon la revendication 7, dans lequel le support solide est choisi dans le groupe constitué par des billes, des billes à code spectral et un microréseau à base de puces.

9. Procédé selon la revendication 6, dans lequel l'étape d'amplification est conduite par PCR en utilisant un ensemble d'amorces d'amplification par PCR, ledit ensemble d'amorces d'amplification par PCR comprenant au moins deux paires d'amorces de PCR choisies dans le groupe des paires constitué par :
SEQ ID NO : 2 et SEQ ID NO : 3, SEQ ID NO : 4 et SEQ ID NO : 5, SEQ ID NO : 6 et SEQ ID NO : 7, et SEQ ID NO : 8 et SEQ ID NO : 9, où les paires d'amorces de PCR sont choisies selon la relation de chaque paire d'amorces avec les variants sélectionnés à détecter.

10. Kit de détection dans un échantillon de la présence ou de l'absence de variants nucléotidiques au niveau de sites polymorphes dans le gène codant pour le cytochrome P450-2D6, ledit kit comprenant un ensemble d'au moins deux amorces d'extension marquées spécifiques d'allèle où chaque amorce d'extension marquée spécifique de l'allèle a une partie hybridante à l'extrémité 3' y compris un nucléotide terminal en 3' soit complémentaire d'un nucléotide variant ou soit du nucléotide de type sauvage correspondant de l'un des sites polymorphes et une partie marqueur à l'extrémité 5' complémentaire d'une séquence anti-marqueur complémentaire, et où les au moins deux amorces d'extension marquées spécifiques d'allèle sont choisies dans le groupe constitué par SEQ ID NO : 10 à SEQ ID NO : 35.

11. Kit selon la revendication 10, comprenant en outre un ensemble d'amorces d'amplification par PCR pour amplifier les régions d'ADN contenant les sites polymorphes, ledit ensemble d'amorces d'amplification par PCR comprenant au moins deux paires d'amorces de PCR choisies dans le groupe de paires constitué par :
SEQ ID NO : 2 et SEQ ID NO : 3, SEQ ID NO : 4 et SEQ ID NO : 5, SEQ ID NO : 6 et SEQ ID NO : 7, et SEQ ID NO : 8 et SEQ ID NO : 9, où les paires d'amorces de PCR sont choisies selon la relation de chaque paire d'amorces avec les variants sélectionnés à détecter.

12. Kit selon la revendication 10, comprenant en outre un ensemble d'anti-marqueurs, chaque anti-marqueur étant complémentaire des nucléotides 1 à 24 des au moins deux amorces d'extension marquées spécifiques d'allèle choisies.

13. Kit selon la revendication 12, dans lequel les anti-marqueurs sont couplés à un support.


### SEQUENCE LISTING

<110> Merante, Frank
   Bortolin, Susan
   Gordon, James D.
   TM Bioscience Corporation
<120> Method of Detecting Mutations in the Gene Encoding cytochrome P450-2D6
<130> 53436/158
<150> U.S. 60/583,605
   <151> June 30, 2004
<160> 35
<170> PatentIn version 3.2
<210> 1
   <211> 9432
   <212> DNA
   <213> Homo sapiens
<300>
   <301> Kimura, S., Umeno, M., Skoda, R.C., Meyer, U.A. and Gonzalez, F.J.
   <302> The human debrisoquine 4-hydroxylase (CYP2D) locus: sequence and idetification of the polymorphic CYP2D6 gene, a related gene, and a pseudogene
   <303> American Journal of Human Genetics
   <304> 45
   <305> 6
   <306> 889-904
   <307> 1989
   <308> M33388.1
   <309> 2004-11-22
   <313> (1)..(9432)
<300>
   <308> M33388.1
   <309> 2004-11-22
   <313> (1)..(9432)
<400> 1
   gaattcaaga ccagcctgga caacttggaa gaacccggtc tctacaaaaa atacaaaatt   60
   agctgggatt gggtgcggtg gctcatgcct ataatcccag cactttggga gcctgaggtg  120
   ggtggatcac ctgaagtcag gagttcaaga ctagcctggc caacatggtg aaaccctatc  180
   tctactgaaa atacaaaaag ctagacgtgg tggcacacac ctgtaatccc agctacttag  240
   gaggctgagg caggagaatt gcttgaagcc tagaggtgaa ggttgtagtg agccgagatt  300
   gcatcattgc acaatggagg ggagccacca gcctgggcaa caagaggaaa tctccgtctc  360
   caaaaaaaaa aaaaaaaaaa aaagaattag gctgggtggt gcctgtagtc ccagctactt  420
   gggaggcagg gggtccactt gatgtcgaga ctgcagtgag ccatgatcct gccactgcac  480
   tccggcctgg gcaacagagt gagaccctgt ctaaagaaaa aaaaaataaa gcaacatatc  540
   ctgaacaaag gatcctccat aacgttccca ccagatttct aatcagaaac atggaggcca  600
   gaaagcagtg gaggaggacg accctcaggc agcccgggag gatgttgtca caggctgggg  660
   caagggcctt ccggctacca actgggagct ctgggaacag ccctgttgca aacaagaagc  720
   catagcccgg ccagagccca ggaatgtggg ctgggctggg agcagcctct ggacaggagt  780
   ggtcccatcc aggaaacctc cggcatggct gggaagtggg gtacttggtg ccgggtctgt  840
   atgtgtgtgt gactggtgtg tgtgagagag aatgtgtgcc ctaagtgtca gtgtgagtct  900
   gtgtatgtgt gaatattgtc tttgtgtggg tgattttctg cgtgtgtaat cgtgtccctg  960
   caagtgtgaa caagtggaca agtgtctggg agtggacaag agatctgtgc accatcaggt 1020
   gtgtgcatag cgtctgtgca tgtcaagagt gcaaggtgaa gtgaagggac caggcccatg 1080
   atgccactca tcatcaggag ctctaaggcc ccaggtaagt gccagtgaca gataagggtg 1140
   ctgaaggtca ctctggagtg ggcaggtggg ggtagggaaa gggcaaggcc atgttctgga 1200
   ggaggggttg tgactacatt agggtgtatg agcctagctg ggaggtggat ggccgggtcc 1260
   actgaaaccc tggttatccc agaaggcttt gcaggcttca ggagcttgga gtggggagag 1320
   ggggtgactt ctccgaccag gcccctccac cggcctaccc tgggtaaggg cctggagcag 1380
   gaagcagggg caagaacctc tggagcagcc catacccgcc ctggcctgac tctgccactg 1440
   gcagcacagt caacacagca ggttcactca cagcagaggg caaaggccat catcagctcc 1500
   ctttataagg gaagggtcac gcgctcggtg tgctgagagt gtcctgcctg gtcctctgtg 1560
   cctggtgggg tgggggtgcc aggtgtgtcc agaggagccc atttggtagt gaggcaggta 1620
   tggggctaga agcactggtg cccctggccg tgatagtggc catcttcctg ctcctggtgg 1680
   acctgatgca ccggcgccaa cgctgggctg cacgctaccc accaggcccc ctgccactgc 1740
   ccgggctggg caacctgctg catgtggact tccagaacac accatactgc ttcgaccagg 1800
   tgagggagga ggtcctggag ggcggcagag gtgctgaggc tcccctacca gaagcaaaca 1860
   tggatggtgg gtgaaaccac aggctggacc agaagccagg ctgagaaggg gaagcaggtt 1920
   tgggggacgt cctggagaag ggcatttata catggcatga aggactggat tttccaaagg 1980
   ccaaggaaga gtagggcaag ggcctggagg tggagctgga cttggcagtg ggcatgcaag 2040
   cccattgggc aacatatgtt atggagtaca aagtcccttc tgctgacacc agaaggaaag 2100
   gccttgggaa tggaagatga gttagtcctg agtgccgttt aaatcacgaa atcgaggatg 2160
   aagggggtgc agtgacccgg ttcaaacctt ttgcactgtg ggtcctcggg cctcactgcc 2220
   tcaccggcat ggaccatcat ctgggaatgg gatgctaact ggggcctctc ggcaattttg 2280
   gtgactcttg caaggtcata cctgggtgac gcatccaaac tgagttcctc catcacagaa 2340
   ggtgtgaccc ccacccccgc cccacgatca ggaggctggg tctcctcctt ccacctgctc 2400
   actcctggta gccccggggg tcgtccaagg ttcaaatagg actaggacct gtagtctggg 2460
   gtgatcctgg cttgacaaga ggccctgacc ctccctctgc agttgcggcg ccgcttcggg 2520
   gacgtgttca gcctgcagct ggcctggacg ccggtggtcg tgctcaatgg gctggcggcc 2580
   gtgcgcgagg cgctggtgac ccacggcgag gacaccgccg accgcccgcc tgtgcccatc 2640
   acccagatcc tgggtttcgg gccgcgttcc caaggcaagc agcggtgggg acagagacag 2700
   atttccgtgg gacccgggtg ggtgatgacc gtagtccgag ctgggcagag agggcgcggg 2760
   gtcgtggaca tgaaacaggc cagcgagtgg ggacagcggg ccaagaaacc acctgcacta 2820
   gggaggtgtg agcatgggga cgagggcggg gcttgtgacg agtgggcggg gccactgccg 2880
   agacctggca ggagcccaat gggtgagcgt ggcgcatttc ccagctggaa tccggtgtcg 2940
   aagtgggggc ggggaccgca cctgtgctgt aagctcagtg tgggtggcgc ggggcccgcg 3000
   gggtcttccc tgagtgcaaa ggcggtcagg gtgggcagag acgaggtggg gcaaagcctg 3060
   ccccagccaa gggagcaagg tggatgcaca aagagtgggc cctgtgacca gctggacaga 3120
   gccagggact gcgggagacc agggggagca tagggttgga gtgggtggtg gatggtgggg 3180
   ctaatgcctt catggccacg cgcacgtgcc cgtcccaccc ccaggggtgt tcctggcgcg 3240
   ctatgggccc gcgtggcgcg agcagaggcg cttctccgtg tccaccttgc gcaacttggg 3300
   cctgggcaag aagtcgctgg agcagtgggt gaccgaggag gccgcctgcc tttgtgccgc 3360
   cttcgccaac cactccggtg ggtgatgggc agaagggcac aaagcgggaa ctgggaaggc 3420
   gggggacggg gaaggcgacc ccttacccgc atctcccacc cccaggacgc ccctttcgcc 3480
   ccaacggtct cttggacaaa gccgtgagca acgtgatcgc ctccctcacc tgcgggcgcc 3540
   gcttcgagta cgacgaccct cgcttcctca ggctgctgga cctagctcag gagggactga 3600
   aggaggagtc gggctttctg cgcgaggtgc ggagcgagag accgaggagt ctctgcaggg 3660
   cgagctcccg agaggtgccg gggctggact ggggcctcgg aagagcagga tttgcataga 3720
   tgggtttggg aaaggacatt ccaggagacc ccactgtaag aagggcctgg aggaggaggg 3780
   gacatctcag acatggtcgt gggagaggtg tgcccgggtc agggggcacc aggagaggcc 3840
   aaggactctg tacctcctat ccacgtcaga gatttcgatt ttaggtttct cctctgggca 3900
   aggagagagg gtggaggctg gcacttgggg agggacttgg tgaggtcagt ggtaaggaca 3960
   ggcaggccct gggtctacct ggagatggct ggggcctgag acttgtccag gtgaacgcag 4020
   agcacaggag ggattgagac cccgttctgt ctggtgtagg tgctgaatgc tgtccccgtc 4080
   ctcctgcata tcccagcgct ggctggcaag gtcctacgct tccaaaaggc tttcctgacc 4140
   cagctggatg agctgctaac tgagcacagg atgacctggg acccagccca gcccccccga 4200
   gacctgactg aggccttcct ggcagagatg gagaaggtga gagtggctgc cacggtgggg 4260
   ggcaagggtg gtgggttgag cgtcccagga ggaatgaggg gaggctgggc aaaaggttgg 4320
   accagtgcat cacccggcga gccgcatctg ggctgacagg tgcagaattg gaggtcattt 4380
   gggggctacc ccgttctgtc ccgagtatgc tctcggccct gctcaggcca aggggaaccc 4440
   tgagagcagc ttcaatgatg agaacctgcg catagtggtg gctgacctgt tctctgccgg 4500
   gatggtgacc acctcgacca cgctggcctg gggcctcctg ctcatgatcc tacatccgga 4560
   tgtgcagcgt gagcccatct gggaaacagt gcaggggccg agggaggaag ggtacaggcg 4620
   ggggcccatg aactttgctg ggacacccgg ggctccaagc acaggcttga ccaggatcct 4680
   gtaagcctga cctcctccaa cataggaggc aagaaggagt gtcagggccg gaccccctgg 4740
   gtgctgaccc attgtgggga cgcatgtctg tccaggccgt gtccaacagg agatcgacga 4800
   cgtgataggg caggtgcggc gaccagagat gggtgaccag gctcacatgc cctacaccac 4860
   tgccgtgatt catgaggtgc agcgctttgg ggacatcgtc cccctgggtg tgacccatat 4920
   gacatcccgt gacatcgaag tacagggctt ccgcatccct aaggtaggcc tggcgccctc 4980
   ctcaccccag ctcagcacca gcacctggtg atagccccag catggctact gccaggtggg 5040
   cccactctag gaaccctggc cacctagtcc tcaatgccac cacactgact gtccccactt 5100
   gggtgggggg tccagagtat aggcagggct ggcctgtcca tccagagccc ccgtctagtg 5160
   gggagacaaa ccaggacctg ccagaatgtt ggaggaccca acgcctgcag ggagaggggg 5220
   cagtgtgggt gcctctgaga ggtgtgactg cgccctgctg tggggtcgga gagggtactg 5280
   tggagcttct cgggcgcagg actagttgac agagtccagc tgtgtgccag gcagtgtgtg 5340
   tcccccgtgt gtttggtggc aggggtccca gcatcctaga gtccagtccc cactctcacc 5400
   ctgcatctcc tgcccaggga acgacactca tcaccaacct gtcatcggtg ctgaaggatg 5460
   aggccgtctg ggagaagccc ttccgcttcc accccgaaca cttcctggat gcccagggcc 5520
   actttgtgaa gccggaggcc ttcctgcctt tctcagcagg tgcctgtggg gagcccggct 5580
   ccctgtcccc ttccgtggag tcttgcaggg gtatcaccca ggagccaggc tcactgacgc 5640
   ccctcccctc cccacaggcc gccgtgcatg cctcggggag cccctggccc gcatggagct 5700
   cttcctcttc ttcacctccc tgctgcagca cttcagcttc tcggtgccca ctggacagcc 5760
   ccggcccagc caccatggtg tctttgcttt cctggtgagc ccatccccct atgagctttg 5820
   tgctgtgccc cgctagaatg gggtacctag tccccagcct gctccctagc cagaggctct 5880
   aatgtacaat aaagcaatgt ggtagttcca actcgggtcc cctgctcacg ccctcgttgg 5940
   gatcatcctc ctcagggcaa ccccacccct gcctcattcc tgcttacccc accgcctggc 6000
   cgcatttgag acaggggtac gttgaggctg agcagatgtc agttaccctt gcccataatc 6060
   ccatgtcccc cactgaccca actctgactg cccagattgg tgacaaggac tacattgtcc 6120
   tggcatgtgg ggaaggggcc agaatgggct gactagaggt gtcagtcagc cctggatgtg 6180
   gtggagaggg caggactcag cctggaggcc catatttcag gcctaactca gcccacccca 6240
   catcagggac agcagtcctg ccagcaccat cacaacagtc acctcccttc atatatgaca 6300
   ccccaaaacg gaagacaaat catggcgtca gggagctata tgccagggct acctacctcc 6360
   cagggctcag tcggcaggtg ccagaacgtt ccctgggaag gccccatgga agcccaggac 6420
   tgagccacca ccctcagcct cgtcacctca ccacaggact ggctacctct ctgggccctc 6480
   agggatgctg ctgtacagac ccctgaccag tgacgagttc gcactcaggg ccaggctggc 6540
   gctggaggag gacacttgtt tggctccaac cctaggtacc atcctcccag tagggatcag 6600
   gcagggccca caggcctgcc ctagggacag gagtcaacct tggacccata aggcactggg 6660
   gcgggcagag aaggaggagg tggcatgggc agctgagagc cagagaccct gaccctagtc 6720
   cttgctctgc cattaccccg tgtgaccccg ggcccaccct tccccaccct tccccacccc 6780
   gggcttctgt ttccttctgc caacgagaag gctgcttcac ctgccccgag tcctgtcttc 6840
   ctgctctgcc ttctggggct gtggcccttg ctggcctgga gccccaacca agggcaggga 6900
   ctgctgtcct ccacgtctgt cctcaccgac ataatgggct gggctgggca cacaggcagt 6960
   gcccaagagt ttctaatgag catatgatta cctgagtcct gggcagacct tcttagggaa 7020
   cagcctggga cagagaacca cagacactct gaggagccac cctgaggcct cttttgccag 7080
   aggaccctac agcctccctg gcagcagttc cgccagcatt tctgtaaatg ccctcatgcc 7140
   agggtgcggc ccggctgtca gcacgagagg gacgttggtc tgtcccctgg caccgagtca 7200
   gtcagaaggg tggccagggc ccccttgggc ccctccagag acaatccact gtggtcacac 7260
   ggctcggtgg caggaagtgc tgttcctgca gctgtgggga cagggagtgt ggatgaagcc 7320
   aggctgggtt tgtctgaaga cggaggcccc gaaaggtggc agcctggcct atagcagcag 7380
   caactcttgg atttattgga aagattttct tcacggttct gagtcttggg ggtgttagag 7440
   gctcagaacc agtccagcca gagctctgtc atgggcacgt agacccggtc ccagggcctt 7500
   tgctctttgc tgtcctcaga ggcctctgca aagtagaaac aggcagcctt gtgagtcccc 7560
   tcctgggagc aaccaaccct ccctctgaga tgccccgggg ccaggtcagc tgtggtgaaa 7620
   ggtagggatg cagccagctc agggagtggc ccagagttcc tgcccaccca aggaggctcc 7680
   caggaaggtc aaggcacctg actcctgggc tgcttccctc ccctcccctc cccaggtcag 7740
   gaaggtggga aagggctggg gtgtctgtga ccctggcagt cactgagaag cagggtggaa 7800
   gcagccccct gcagcacgct gggtcagtgg tcttaccaga tggatacgca gcaacttcct 7860
   tttgaacctt tttattttcc tggcaggaag aagagggatc cagcagtgag atcaggcagg 7920
   ttctgtgttg cacagacagg gaaacaggct ctgtccacac aaagtcggtg gggccaggat 7980
   gaggcccagt ctgttcacac atggctgctg cctctcagct ctgcacagac gtcctcgctc 8040
   ccctgggatg gcagcttggc ctgctggtct tggggttgag ccagcctcca gcactgcctc 8100
   cctgccctgc tgcctcccac tctgcagtgc tccatggctg ctcagttgga cccacgctgg 8160
   agacgttcag tcgaagcccc gggctgtcct tacctcccag tctggggtac ctgccacctc 8220
   ctgctcagca ggaatggggc taggtgcttc ctcccctggg gacttcacct gctctccctc 8280
   ctgggataag acggcagcct cctccttggg ggcagcagca ttcagtcctc caggtctcct 8340
   gggggtcgtg acctgcagga ggaataagag ggcagactgg gcagaaaggc cttcagagca 8400
   cctcatcctc ctgttctcac actggggtgt cacagtcctg ggaagttctt ccttttcagt 8460
   tgagctgtgg taaccttgtg agtttcctgg agggggcctg ccactaccct tgggactccc 8520
   tgccgtgtgt ctgggtctaa ctgagctctg aaaggagaga gccccagccc tgggccttcc 8580
   aggggaagcc ttacctcaga ggttggcttc ttcctactct tgactttgcg tctctgcaga 8640
   gggaggtggg aggggtgaca caaccctgac acccacacta tgagtgatga gtagtcctgc 8700
   cccgactggc ccatcctttc caggtgcagt cccccttact gtgtctgcca agggtgccag 8760
   cacagccgcc ccactccagg ggaagaggag tgccagccct taccacctga gtgggcacag 8820
   tgtagcattt attcattagc ccccacactg gcctgaccat ctcccctgtg ggctgcatga 8880
   caaggagaga gaacaggctg aggtgagagc tactgtcaac acctaaacct aaaaaatcta 8940
   taattgggct gggcagggtg gctcacgcct gtaatcccag cactttggga ggccgagatg 9000
   ggtggatcac ctgaggtcag atgttcgaga ccagcctggc caacatggtg aaaccccgtc 9060
   tctactaaaa atacaaaaaa ttagctgggc gtggtggtgg gtgcctgtaa tcccagctac 9120
   tcaggaggct gaggcaggag aattgcttga acctgggagg cagaggctgc agtgagccga 9180
   gatcgcatca ttgcactcca gcctggtcaa caagagtgaa actgtcttaa aaaaaaaatc 9240
   tataattgat atctttagaa agataaaact ttgcattcat gaaataagaa taggagggtc 9300
   taaaataaaa atgttcaaac acccaccacc actaattctt gacaaaaata tagtctgggt 9360
   gccttagctc atgcctgtaa tcccagcatt ttgggaggct aaggcaggag gattgtttga 9420
   gcctaggaat tc 9432
<210> 2
   <211> 19
   <212> DNA
   <213> Artificial
<220>
   <223> FORWARD PRIMER
<400> 2
   actggctcca agcatggca 19
<210> 3
   <211> 19
   <212> DNA
   <213> Artificial
<220>
   <223> REVERSE PRIMER
<400> 3
   tcgccctgca gagactcct 19
<210> 4
   <211> 20
   <212> DNA
   <213> Artificial
<220>
   <223> FORWARD PRIMER
<400> 4
   cctcagcctc gtcacctcac 20
<210> 5
   <211> 20
   <212> DNA
   <213> Artificial
<220>
   <223> REVERSE PRIMER
<400> 5
   cacgtgcagg gcacctagat 20
<210> 6
   <211> 23
   <212> DNA
   <213> Artificial
<220>
   <223> FORWARD PRIMER
<400> 6
   ggccaaggac tctgtacctc cta 23
<210> 7
   <211> 21
   <212> DNA
   <213> Artificial
<220>
   <223> REVERSE PRIMER
<400> 7
   cagtcctgtg gtgaggtgac g 21
<210> 8
   <211> 21
   <212> DNA
   <213> Artificial
<220>
   <223> FORWARD PRIMER
<400> 8
   accgggcacc tgtactcctc a 21
<210> 9
   <211> 22
   <212> DNA
   <213> Artificial
<220>
   <223> REVERSE PRIMER
<400> 9
   gcatgagcta aggcacccag ac 22
<210> 10
   <211> 46
   <212> DNA
   <213> Artificial
<220>
   <223> ASPE PRIMER
<400> 10
   tacaacatct cattaacata tacacctgga caacttggaa gaaccc 46
<210> 11
   <211> 48
   <212> DNA
   <213> Artificial
<220>
   <223> ASPE PRIMER
<400> 11
   ttaaacaatc tactattcaa tcacagcctg gacaacttgg aagaaccg 48
<210> 12
   <211> 44
   <212> DNA
   <213> Artificial
<220>
   <223> ASPE PRIMER
<400> 12
   acatcaaatt ctttcaatat cttctgagct gctaactgag caca 44
<210> 13
   <211> 44
   <212> DNA
   <213> Artificial
<220>
   <223> ASPE PRIMER
<400> 13
   taatttcttt catctctaca taactgagct gctaactgag cacg 44
<210> 14
   <211> 43
   <212> DNA
   <213> Artificial
<220>
   <223> ASPE PRIMER
<400> 14
   aatcaacaca caataacatt catatggggc gaaaggggcg tcc 43
<210> 15
   <211> 43
   <212> DNA
   <213> Artificial
<220>
   <223> ASPE PRIMER
<400> 15
   aataacaact cactatatca taactggggc gaaaggggcg tct 43
<210> 16
   <211> 43
   <212> DNA
   <213> Artificial
<220>
   <223> ASPE PRIMER
<400> 16
   acaaatatct aactactatc acaacgctgg gctgcacgct acc 43
<210> 17
   <211> 43
   <212> DNA
   <213> Artificial
<220>
   <223> ASPE PRIMER
<400> 17
   taaatcaata ctcataatct cactcgctgg gctgcacgct act 43
<210> 18
   <211> 43
   <212> DNA
   <213> Artificial
<220>
   <223> ASPE PRIMER
<400> 18
   atctcaatta caataacaca caaacggcct cctcggtcac cca 43
<210> 19
   <211> 45
   <212> DNA
   <213> Artificial
<220>
   <223> ASPE PRIMER
<400> 19
   tacacaatat tcatcataac taacggcggc ctcctcggtc acccc 45
<210> 20
   <211> 45
   <212> DNA
   <213> Artificial
<220>
   <223> ASPE PRIMER
<400> 20
   caatttacat ttcactttct tatccctcct gctcatgatc ctaca 45
<210> 21
   <211> 45
   <212> DNA
   <213> Artificial
<220>
   <223> ASPE PRIMER
<400> 21
   cttaaactct acttacttct aattcctcct gctcatgatc ctacc 45
<210> 22
   <211> 44
   <212> DNA
   <213> Artificial
<220>
   <223> ASPE PRIMER
<400> 22
   tcatcacttt ctttacttta cattcgcctt cgccaaccac tccg 44
<210> 23
   <211> 44
   <212> DNA
   <213> Artificial
<220>
   <223> ASPE PRIMER
<400> 23
   tcacttatta cttcacatta tctacgcctt cgccaaccac tcct 44
<210> 24
   <211> 42
   <212> DNA
   <213> Artificial
<220>
   <223> ASPE PRIMER
<400> 24
   atatacttta cactttcaac aaacctgacc ctccctctgc ag 42
<210> 25
   <211> 42
   <212> DNA
   <213> Artificial
<220>
   <223> ASPE PRIMER
<400> 25
   tacttcttta ctacaattta caacctgacc ctccctctgc ac 42
<210> 26
   <211> 44
   <212> DNA
   <213> Artificial
<220>
   <223> ASPE PRIMER
<400> 26
   tacttaaaca tacaaactta ctcatgcagc aggttgccca gccc 44
<210> 27
   <211> 44
   <212> DNA
   <213> Artificial
<220>
   <223> ASPE PRIMER
<400> 27
   ctatcattta tctctttctc aatttgcagc aggttgccca gcct 44
<210> 28
   <211> 45
   <212> DNA
   <213> Artificial
<220>
   <223> ASPE PRIMER
<400> 28
   atactttaca aacaaataac acacagcttc aatgatgaga acctg 45
<210> 29
   <211> 44
   <212> DNA
   <213> Artificial
<220>
   <223> ASPE PRIMER
<400> 29
   ctacaaacac ttaactttat ctttcttcaa tgatgagaac ctgt 44
<210> 30
   <211> 42
   <212> DNA
   <213> Artificial
<220>
   <223> ASPE PRIMER
<400> 30
   cttaacattt aacttctata acacggcagc cactctcacc tt 42
<210> 31
   <211> 42
   <212> DNA
   <213> Artificial
<220>
   <223> ASPE PRIMER
<400> 31
   cacaacaaat tcttattcaa ttcaggcagc cactctcacc tc 42
<210> 32
   <211> 42
   <212> DNA
   <213> Artificial
<220>
   <223> ASPE PRIMER
<400> 32
   aactttctct ctctattctt atttatggcg tttcatactt at 42
<210> 33
   <211> 43
   <212> DNA
   <213> Artificial
<220>
   <223> ASPE PRIMER
<400> 33
   cacttaattc attctaaatc tatccgccag cacgttgaca cct 43
<210> 34
   <211> 46
   <212> DNA
   <213> Artificial
<220>
   <223> ASPE PRIMER
<400> 34
   ttcaaacatt caaattacta ctacaccgcc cgcctgtgcc catcac 46
<210> 35
   <211> 44
   <212> DNA
   <213> Artificial
<220>
   <223> ASPE PRIMER
<400> 35
   ttaatacaat tctctctttc tctacgcccg cctgtgccca tcat 44

### SEQUENCE LISTING

<110> TM Bioscience PGX, Inc.
<120> Method of Detecting Mutations in the Gene Encoding Cytochrome P450-2D6
<130> P027846EP
<140> 05761958.7
   <141> 2005-06-30
<150> US 60/583,605
   <151> 2004-06-30
<160> 35
<170> PatentIn version 3.2
<210> 1
   <211> 9432
   <212> DNA
   <213> Homo sapiens
<300>
   <301> Kimura, S., Umeno, M., Skoda, R.C., Meyer, U.A. and Gonzalez, F.J.
   <302> The human debrisoquine 4-hydroxylase (CYP2D) locus: sequence and identification of the polymorphic CYP2D6 gene, a related gene, and a pseudogene
   <303> American Journal of Human Genetics
   <304> 45
   <305> 6
   <306> 889-904
   <307> 1989
   <308> M33388.1
   <309> 2004-11-22
   <313> (1)..(9432)
<300>
   <308> M33388.1
   <309> 2004-11-22
   <313> (1)..(9432)
<400> 1
   gaattcaaga ccagcctgga caacttggaa gaacccggtc tctacaaaaa atacaaaatt    60
   agctgggatt gggtgcggtg gctcatgcct ataatcccag cactttggga gcctgaggtg   120
   ggtggatcac ctgaagtcag gagttcaaga ctagcctggc caacatggtg aaaccctatc   180
   tctactgaaa atacaaaaag ctagacgtgg tggcacacac ctgtaatccc agctacttag   240
   gaggctgagg caggagaatt gcttgaagcc tagaggtgaa ggttgtagtg agccgagatt   300
   gcatcattgc acaatggagg ggagccacca gcctgggcaa caagaggaaa tctccgtctc   360
   caaaaaaaaa aaaaaaaaaa aaagaattag gctgggtggt gcctgtagtc ccagctactt   420
   gggaggcagg gggtccactt gatgtcgaga ctgcagtgag ccatgatcct gccactgcac   480
   tccggcctgg gcaacagagt gagaccctgt ctaaagaaaa aaaaaataaa gcaacatatc   540
   ctgaacaaag gatcctccat aacgttccca ccagatttct aatcagaaac atggaggcca   600
   gaaagcagtg gaggaggacg accctcaggc agcccgggag gatgttgtca caggctgggg  660
   caagggcctt ccggctacca actgggagct ctgggaacag ccctgttgca aacaagaagc  720
   catagcccgg ccagagccca ggaatgtggg ctgggctggg agcagcctct ggacaggagt  780
   ggtcccatcc aggaaacctc cggcatggct gggaagtggg gtacttggtg ccgggtctgt  840
   atgtgtgtgt gactggtgtg tgtgagagag aatgtgtgcc ctaagtgtca gtgtgagtct  900
   gtgtatgtgt gaatattgtc tttgtgtggg tgattttctg cgtgtgtaat cgtgtccctg  960
   caagtgtgaa caagtggaca agtgtctggg agtggacaag agatctgtgc accatcaggt 1020
   gtgtgcatag cgtctgtgca tgtcaagagt gcaaggtgaa gtgaagggac caggcccatg 1080
   atgccactca tcatcaggag ctctaaggcc ccaggtaagt gccagtgaca gataagggtg 1140
   ctgaaggtca ctctggagtg ggcaggtggg ggtagggaaa gggcaaggcc atgttctgga 1200
   ggaggggttg tgactacatt agggtgtatg agcctagctg ggaggtggat ggccgggtcc 1260
   actgaaaccc tggttatccc agaaggcttt gcaggcttca ggagcttgga gtggggagag 1320
   ggggtgactt ctccgaccag gcccctccac cggcctaccc tgggtaaggg cctggagcag 1380
   gaagcagggg caagaacctc tggagcagcc catacccgcc ctggcctgac tctgccactg 1440
   gcagcacagt caacacagca ggttcactca cagcagaggg caaaggccat catcagctcc 1500
   ctttataagg gaagggtcac gcgctcggtg tgctgagagt gtcctgcctg gtcctctgtg 1560
   cctggtgggg tgggggtgcc aggtgtgtcc agaggagccc atttggtagt gaggcaggta 1620
   tggggctaga agcactggtg cccctggccg tgatagtggc catcttcctg ctcctggtgg 1680
   acctgatgca ccggcgccaa cgctgggctg cacgctaccc accaggcccc ctgccactgc 1740
   ccgggctggg caacctgctg catgtggact tccagaacac accatactgc ttcgaccagg 1800
   tgagggagga ggtcctggag ggcggcagag gtgctgaggc tcccctacca gaagcaaaca 1860
   tggatggtgg gtgaaaccac aggctggacc agaagccagg ctgagaaggg gaagcaggtt 1920
   tgggggacgt cctggagaag ggcatttata catggcatga aggactggat tttccaaagg 1980
   ccaaggaaga gtagggcaag ggcctggagg tggagctgga cttggcagtg ggcatgcaag 2040
   cccattgggc aacatatgtt atggagtaca aagtcccttc tgctgacacc agaaggaaag 2100
   gccttgggaa tggaagatga gttagtcctg agtgccgttt aaatcacgaa atcgaggatg 2160
   aagggggtgc agtgacccgg ttcaaacctt ttgcactgtg ggtcctcggg cctcactgcc 2220
   tcaccggcat ggaccatcat ctgggaatgg gatgctaact ggggcctctc ggcaattttg 2280
   gtgactcttg caaggtcata cctgggtgac gcatccaaac tgagttcctc catcacagaa 2340
   ggtgtgaccc ccacccccgc cccacgatca ggaggctggg tctcctcctt ccacctgctc 2400
   actcctggta gccccggggg tcgtccaagg ttcaaatagg actaggacct gtagtctggg 2460
   gtgatcctgg cttgacaaga ggccctgacc ctccctctgc agttgcggcg ccgcttcggg 2520
   gacgtgttca gcctgcagct ggcctggacg ccggtggtcg tgctcaatgg gctggcggcc 2580
   gtgcgcgagg cgctggtgac ccacggcgag gacaccgccg accgcccgcc tgtgcccatc 2640
   acccagatcc tgggtttcgg gccgcgttcc caaggcaagc agcggtgggg acagagacag 2700
   atttccgtgg gacccgggtg ggtgatgacc gtagtccgag ctgggcagag agggcgcggg 2760
   gtcgtggaca tgaaacaggc cagcgagtgg ggacagcggg ccaagaaacc acctgcacta 2820
   gggaggtgtg agcatgggga cgagggcggg gcttgtgacg agtgggcggg gccactgccg 2880
   agacctggca ggagcccaat gggtgagcgt ggcgcatttc ccagctggaa tccggtgtcg 2940
   aagtgggggc ggggaccgca cctgtgctgt aagctcagtg tgggtggcgc ggggcccgcg 3000
   gggtcttccc tgagtgcaaa ggcggtcagg gtgggcagag acgaggtggg gcaaagcctg 3060
   ccccagccaa gggagcaagg tggatgcaca aagagtgggc cctgtgacca gctggacaga 3120
   gccagggact gcgggagacc agggggagca tagggttgga gtgggtggtg gatggtgggg 3180
   ctaatgcctt catggccacg cgcacgtgcc cgtcccaccc ccaggggtgt tcctggcgcg 3240
   ctatgggccc gcgtggcgcg agcagaggcg cttctccgtg tccaccttgc gcaacttggg 3300
   cctgggcaag aagtcgctgg agcagtgggt gaccgaggag gccgcctgcc tttgtgccgc 3360
   cttcgccaac cactccggtg ggtgatgggc agaagggcac aaagcgggaa ctgggaaggc 3420
   gggggacggg gaaggcgacc ccttacccgc atctcccacc cccaggacgc ccctttcgcc 3480
   ccaacggtct cttggacaaa gccgtgagca acgtgatcgc ctccctcacc tgcgggcgcc 3540
   gcttcgagta cgacgaccct cgcttcctca ggctgctgga cctagctcag gagggactga 3600
   aggaggagtc gggctttctg cgcgaggtgc ggagcgagag accgaggagt ctctgcaggg 3660
   cgagctcccg agaggtgccg gggctggact ggggcctcgg aagagcagga tttgcataga 3720
   tgggtttggg aaaggacatt ccaggagacc ccactgtaag aagggcctgg aggaggaggg 3780
   gacatctcag acatggtcgt gggagaggtg tgcccgggtc agggggcacc aggagaggcc 3840
   aaggactctg tacctcctat ccacgtcaga gatttcgatt ttaggtttct cctctgggca 3900
   aggagagagg gtggaggctg gcacttgggg agggacttgg tgaggtcagt ggtaaggaca 3960
   ggcaggccct gggtctacct ggagatggct ggggcctgag acttgtccag gtgaacgcag 4020
   agcacaggag ggattgagac cccgttctgt ctggtgtagg tgctgaatgc tgtccccgtc 4080
   ctcctgcata tcccagcgct ggctggcaag gtcctacgct tccaaaaggc tttcctgacc 4140
   cagctggatg agctgctaac tgagcacagg atgacctggg acccagccca gcccccccga 4200
   gacctgactg aggccttcct ggcagagatg gagaaggtga gagtggctgc cacggtgggg 4260
   ggcaagggtg gtgggttgag cgtcccagga ggaatgaggg gaggctgggc aaaaggttgg 4320
   accagtgcat cacccggcga gccgcatctg ggctgacagg tgcagaattg gaggtcattt 4380
   gggggctacc ccgttctgtc ccgagtatgc tctcggccct gctcaggcca aggggaaccc 4440
   tgagagcagc ttcaatgatg agaacctgcg catagtggtg gctgacctgt tctctgccgg 4500
   gatggtgacc acctcgacca cgctggcctg gggcctcctg ctcatgatcc tacatccgga 4560
   tgtgcagcgt gagcccatct gggaaacagt gcaggggccg agggaggaag ggtacaggcg 4620
   ggggcccatg aactttgctg ggacacccgg ggctccaagc acaggcttga ccaggatcct 4680
   gtaagcctga cctcctccaa cataggaggc aagaaggagt gtcagggccg gaccccctgg 4740
   gtgctgaccc attgtgggga cgcatgtctg tccaggccgt gtccaacagg agatcgacga 4800
   cgtgataggg caggtgcggc gaccagagat gggtgaccag gctcacatgc cctacaccac 4860
   tgccgtgatt catgaggtgc agcgctttgg ggacatcgtc cccctgggtg tgacccatat 4920
   gacatcccgt gacatcgaag tacagggctt ccgcatccct aaggtaggcc tggcgccctc 4980
   ctcaccccag ctcagcacca gcacctggtg atagccccag catggctact gccaggtggg 5040
   cccactctag gaaccctggc cacctagtcc tcaatgccac cacactgact gtccccactt 5100
   gggtgggggg tccagagtat aggcagggct ggcctgtcca tccagagccc ccgtctagtg 5160
   gggagacaaa ccaggacctg ccagaatgtt ggaggaccca acgcctgcag ggagaggggg 5220
   cagtgtgggt gcctctgaga ggtgtgactg cgccctgctg tggggtcgga gagggtactg 5280
   tggagcttct cgggcgcagg actagttgac agagtccagc tgtgtgccag gcagtgtgtg 5340
   tcccccgtgt gtttggtggc aggggtccca gcatcctaga gtccagtccc cactctcacc 5400
   ctgcatctcc tgcccaggga acgacactca tcaccaacct gtcatcggtg ctgaaggatg 5460
   aggccgtctg ggagaagccc ttccgcttcc accccgaaca cttcctggat gcccagggcc 5520
   actttgtgaa gccggaggcc ttcctgcctt tctcagcagg tgcctgtggg gagcccggct 5580
   ccctgtcccc ttccgtggag tcttgcaggg gtatcaccca ggagccaggc tcactgacgc 5640
   ccctcccctc cccacaggcc gccgtgcatg cctcggggag cccctggccc gcatggagct 5700
   cttcctcttc ttcacctccc tgctgcagca cttcagcttc tcggtgccca ctggacagcc 5760
   ccggcccagc caccatggtg tctttgcttt cctggtgagc ccatccccct atgagctttg 5820
   tgctgtgccc cgctagaatg gggtacctag tccccagcct gctccctagc cagaggctct 5880
   aatgtacaat aaagcaatgt ggtagttcca actcgggtcc cctgctcacg ccctcgttgg 5940
   gatcatcctc ctcagggcaa ccccacccct gcctcattcc tgcttacccc accgcctggc 6000
   cgcatttgag acaggggtac gttgaggctg agcagatgtc agttaccctt gcccataatc 6060
   ccatgtcccc cactgaccca actctgactg cccagattgg tgacaaggac tacattgtcc 6120
   tggcatgtgg ggaaggggcc agaatgggct gactagaggt gtcagtcagc cctggatgtg 6180
   gtggagaggg caggactcag cctggaggcc catatttcag gcctaactca gcccacccca 6240
   catcagggac agcagtcctg ccagcaccat cacaacagtc acctcccttc atatatgaca 6300
   ccccaaaacg gaagacaaat catggcgtca gggagctata tgccagggct acctacctcc 6360
   cagggctcag tcggcaggtg ccagaacgtt ccctgggaag gccccatgga agcccaggac 6420
   tgagccacca ccctcagcct cgtcacctca ccacaggact ggctacctct ctgggccctc 6480
   agggatgctg ctgtacagac ccctgaccag tgacgagttc gcactcaggg ccaggctggc 6540
   gctggaggag gacacttgtt tggctccaac cctaggtacc atcctcccag tagggatcag 6600
   gcagggccca caggcctgcc ctagggacag gagtcaacct tggacccata aggcactggg 6660
   gcgggcagag aaggaggagg tggcatgggc agctgagagc cagagaccct gaccctagtc 6720
   cttgctctgc cattaccccg tgtgaccccg ggcccaccct tccccaccct tccccacccc 6780
   gggcttctgt ttccttctgc caacgagaag gctgcttcac ctgccccgag tcctgtcttc 6840
   ctgctctgcc ttctggggct gtggcccttg ctggcctgga gccccaacca agggcaggga 6900
   ctgctgtcct ccacgtctgt cctcaccgac ataatgggct gggctgggca cacaggcagt 6960
   gcccaagagt ttctaatgag catatgatta cctgagtcct gggcagacct tcttagggaa 7020
   cagcctggga cagagaacca cagacactct gaggagccac cctgaggcct cttttgccag 7080
   aggaccctac agcctccctg gcagcagttc cgccagcatt tctgtaaatg ccctcatgcc 7140
   agggtgcggc ccggctgtca gcacgagagg gacgttggtc tgtcccctgg caccgagtca 7200
   gtcagaaggg tggccagggc ccccttgggc ccctccagag acaatccact gtggtcacac 7260
   ggctcggtgg caggaagtgc tgttcctgca gctgtgggga cagggagtgt ggatgaagcc 7320
   aggctgggtt tgtctgaaga cggaggcccc gaaaggtggc agcctggcct atagcagcag 7380
   caactcttgg atttattgga aagattttct tcacggttct gagtcttggg ggtgttagag 7440
   gctcagaacc agtccagcca gagctctgtc atgggcacgt agacccggtc ccagggcctt 7500
   tgctctttgc tgtcctcaga ggcctctgca aagtagaaac aggcagcctt gtgagtcccc 7560
   tcctgggagc aaccaaccct ccctctgaga tgccccgggg ccaggtcagc tgtggtgaaa 7620
   ggtagggatg cagccagctc agggagtggc ccagagttcc tgcccaccca aggaggctcc 7680
   caggaaggtc aaggcacctg actcctgggc tgcttccctc ccctcccctc cccaggtcag 7740
   gaaggtggga aagggctggg gtgtctgtga ccctggcagt cactgagaag cagggtggaa 7800
   gcagccccct gcagcacgct gggtcagtgg tcttaccaga tggatacgca gcaacttcct 7860
   tttgaacctt tttattttcc tggcaggaag aagagggatc cagcagtgag atcaggcagg 7920
   ttctgtgttg cacagacagg gaaacaggct ctgtccacac aaagtcggtg gggccaggat 7980
   gaggcccagt ctgttcacac atggctgctg cctctcagct ctgcacagac gtcctcgctc 8040
   ccctgggatg gcagcttggc ctgctggtct tggggttgag ccagcctcca gcactgcctc 8100
   cctgccctgc tgcctcccac tctgcagtgc tccatggctg ctcagttgga cccacgctgg 8160
   agacgttcag tcgaagcccc gggctgtcct tacctcccag tctggggtac ctgccacctc 8220
   ctgctcagca ggaatggggc taggtgcttc ctcccctggg gacttcacct gctctccctc 8280
   ctgggataag acggcagcct cctccttggg ggcagcagca ttcagtcctc caggtctcct 8340
   gggggtcgtg acctgcagga ggaataagag ggcagactgg gcagaaaggc cttcagagca 8400
   cctcatcctc ctgttctcac actggggtgt cacagtcctg ggaagttctt ccttttcagt 8460
   tgagctgtgg taaccttgtg agtttcctgg agggggcctg ccactaccct tgggactccc 8520
   tgccgtgtgt ctgggtctaa ctgagctctg aaaggagaga gccccagccc tgggccttcc 8580
   aggggaagcc ttacctcaga ggttggcttc ttcctactct tgactttgcg tctctgcaga 8640
   gggaggtggg aggggtgaca caaccctgac acccacacta tgagtgatga gtagtcctgc 8700
   cccgactggc ccatcctttc caggtgcagt cccccttact gtgtctgcca agggtgccag 8760
   cacagccgcc ccactccagg ggaagaggag tgccagccct taccacctga gtgggcacag 8820
   tgtagcattt attcattagc ccccacactg gcctgaccat ctcccctgtg ggctgcatga 8880
   caaggagaga gaacaggctg aggtgagagc tactgtcaac acctaaacct aaaaaatcta 8940
   taattgggct gggcagggtg gctcacgcct gtaatcccag cactttggga ggccgagatg 9000
   ggtggatcac ctgaggtcag atgttcgaga ccagcctggc caacatggtg aaaccccgtc 9060
   tctactaaaa atacaaaaaa ttagctgggc gtggtggtgg gtgcctgtaa tcccagctac 9120
   tcaggaggct gaggcaggag aattgcttga acctgggagg cagaggctgc agtgagccga 9180
   gatcgcatca ttgcactcca gcctggtcaa caagagtgaa actgtcttaa aaaaaaaatc 9240
   tataattgat atctttagaa agataaaact ttgcattcat gaaataagaa taggagggtc 9300
   taaaataaaa atgttcaaac acccaccacc actaattctt gacaaaaata tagtctgggt 9360
   gccttagctc atgcctgtaa tcccagcatt ttgggaggct aaggcaggag gattgtttga 9420
   gcctaggaat tc 9432
<210> 2
   <211> 19
   <212> DNA
   <213> Artificial
<220>
   <223> FORWARD PRIMER
<400> 2
   actggctcca agcatggca 19
<210> 3
   <211> 19
   <212> DNA
   <213> Artificial
<220>
   <223> REVERSE PRIMER
<400> 3
   tcgccctgca gagactcct 19
<210> 4
   <211> 20
   <212> DNA
   <213> Artificial
<220>
   <223> FORWARD PRIMER
<400> 4
   cctcagcctc gtcacctcac 20
<210> 5
   <211> 20
   <212> DNA
   <213> Artificial
<220>
   <223> REVERSE PRIMER
<400> 5
   cacgtgcagg gcacctagat 20
<210> 6
   <211> 23
   <212> DNA
   <213> Artificial
<220>
   <223> FORWARD PRIMER
<400> 6
   ggccaaggac tctgtacctc cta 23
<210> 7
   <211> 21
   <212> DNA
   <213> Artificial
<220>
   <223> REVERSE PRIMER
<400> 7
   cagtcctgtg gtgaggtgac g 21
<210> 8
   <211> 21
   <212> DNA
   <213> Artificial
<220>
   <223> FORWARD PRIMER
<400> 8
   accgggcacc tgtactcctc a 21
<210> 9
   <211> 22
   <212> DNA
   <213> Artificial
<220>
   <223> REVERSE PRIMER
<400> 9
   gcatgagcta aggcacccag ac 22
<210> 10
   <211> 46
   <212> DNA
   <213> Artificial
<220>
   <223> ASPE PRIMER
<400> 10
   tacaacatct cattaacata tacacctgga caacttggaa gaaccc 46
<210> 11
   <211> 48
   <212> DNA
   <213> Artificial
<220>
   <223> ASPE PRIMER
<400> 11
   ttaaacaatc tactattcaa tcacagcctg gacaacttgg aagaaccg 48
<210> 12
   <211> 44
   <212> DNA
   <213> Artificial
<220>
   <223> ASPE PRIMER
<400> 12
   acatcaaatt ctttcaatat cttctgagct gctaactgag caca 44
<210> 13
   <211> 44
   <212> DNA
   <213> Artificial
<220>
   <223> ASPE PRIMER
<400> 13
   taatttcttt catctctaca taactgagct gctaactgag cacg 44
<210> 14
   <211> 43
   <212> DNA
   <213> Artificial
<220>
   <223> ASPE PRIMER
<400> 14
   aatcaacaca caataacatt catatggggc gaaaggggcg tcc 43
<210> 15
   <211> 43
   <212> DNA
   <213> Artificial
<220>
   <223> ASPE PRIMER
<400> 15
   aataacaact cactatatca taactggggc gaaaggggcg tct 43
<210> 16
   <211> 43
   <212> DNA
   <213> Artificial
<220>
   <223> ASPE PRIMER
<400> 16
   acaaatatct aactactatc acaacgctgg gctgcacgct acc 43
<210> 17
   <211> 43
   <212> DNA
   <213> Artificial
<220>
   <223> ASPE PRIMER
<400> 17
   taaatcaata ctcataatct cactcgctgg gctgcacgct act 43
<210> 18
   <211> 43
   <212> DNA
   <213> Artificial
<220>
   <223> ASPE PRIMER
<400> 18
   atctcaatta caataacaca caaacggcct cctcggtcac cca 43
<210> 19
   <211> 45
   <212> DNA
   <213> Artificial
<220>
   <223> ASPE PRIMER
<400> 19
   tacacaatat tcatcataac taacggcggc ctcctcggtc acccc 45
<210> 20
   <211> 45
   <212> DNA
   <213> Artificial
<220>
   <223> ASPE PRIMER
<400> 20
   caatttacat ttcactttct tatccctcct gctcatgatc ctaca 45
<210> 21
   <211> 45
   <212> DNA
   <213> Artificial
<220>
   <223> ASPE PRIMER
<400> 21
   cttaaactct acttacttct aattcctcct gctcatgatc ctacc 45
<210> 22
   <211> 44
   <212> DNA
   <213> Artificial
<220>
   <223> ASPE PRIMER
<400> 22
   tcatcacttt ctttacttta cattcgcctt cgccaaccac tccg 44
<210> 23
   <211> 44
   <212> DNA
   <213> Artificial
<220>
   <223> ASPE PRIMER
<400> 23
   tcacttatta cttcacatta tctacgcctt cgccaaccac tcct 44
<210> 24
   <211> 42
   <212> DNA
   <213> Artificial
<220>
   <223> ASPE PRIMER
<400> 24
   atatacttta cactttcaac aaacctgacc ctccctctgc ag 42
<210> 25
   <211> 42
   <212> DNA
   <213> Artificial
<220>
   <223> ASPE PRIMER
<400> 25
   tacttcttta ctacaattta caacctgacc ctccctctgc ac 42
<210> 26
   <211> 44
   <212> DNA
   <213> Artificial
<220>
   <223> ASPE PRIMER
<400> 26
   tacttaaaca tacaaactta ctcatgcagc aggttgccca gccc 44
<210> 27
   <211> 44
   <212> DNA
   <213> Artificial
<220>
   <223> ASPE PRIMER
<400> 27
   ctatcattta tctctttctc aatttgcagc aggttgccca gcct 44
<210> 28
   <211> 45
   <212> DNA
   <213> Artificial
<220>
   <223> ASPE PRIMER
<400> 28
   atactttaca aacaaataac acacagcttc aatgatgaga acctg 45
<210> 29
   <211> 44
   <212> DNA
   <213> Artificial
<220>
   <223> ASPE PRIMER
<400> 29
   ctacaaacac ttaactttat ctttcttcaa tgatgagaac ctgt 44
<210> 30
   <211> 42
   <212> DNA
   <213> Artificial
<220>
   <223> ASPE PRIMER
<400> 30
   cttaacattt aacttctata acacggcagc cactctcacc tt 42
<210> 31
   <211> 42
   <212> DNA
   <213> Artificial
<220>
   <223> ASPE PRIMER
<400> 31
   cacaacaaat tcttattcaa ttcaggcagc cactctcacc tc 42
<210> 32
   <211> 42
   <212> DNA
   <213> Artificial
<220>
   <223> ASPE PRIMER
<400> 32
   aactttctct ctctattctt atttatggcg tttcatactt at 42
<210> 33
   <211> 43
   <212> DNA
   <213> Artificial
<220>
   <223> ASPE PRIMER
<400> 33
   cacttaattc attctaaatc tatccgccag cacgttgaca cct 43
<210> 34
   <211> 46
   <212> DNA
   <213> Artificial
<220>
   <223> ASPE PRIMER
<400> 34
   ttcaaacatt caaattacta ctacaccgcc cgcctgtgcc catcac 46
<210> 35
   <211> 44
   <212> DNA
   <213> Artificial
<220>
   <223> ASPE PRIMER
<400> 35
   ttaatacaat tctctctttc tctacgcccg cctgtgccca tcat 44